(19) Europäisches Patentamt
European Patent Office
Office européen des brevets

(11) Veröffentlichungsnummer: **0 255 989**
**B1**

(12) # EUROPÄISCHE PATENTSCHRIFT

(45) Veröffentlichungstag der Patentschrift: **22.11.90**

(51) Int. Cl.⁵: **G03F 7/038**

(21) Anmeldenummer: **87810438.9**

(22) Anmeldetag: **31.07.87**

(54) **Negativ-Photoresist auf Basis von Polyphenolen und Epoxidverbindungen oder Vinylethern.**

(30) Priorität: **06.08.86 CH 3157/86**

(43) Veröffentlichungstag der Anmeldung:
**17.02.88 Patentblatt 88/7**

(45) Bekanntmachung des Hinweises auf die Patenterteilung:
**22.11.90 Patentblatt 90/47**

(84) Benannte Vertragsstaaten:
**CH DE FR GB IT LI NL SE**

(56) Entgegenhaltungen:
**EP-A- 0 118 748**
**EP-A- 0 146 498**
**EP-A- 0 153 682**
**EP-A- 0 153 904**
**EP-A- 0 174 510**
**EP-A- 0 222 187**
**DD-A- 206 518**
**DE-A- 2 322 230**
**DE-A- 2 733 267**
**FR-A- 1 507 529**
**FR-A- 2 310 367**
**JP-A- 6 071 657**
**US-A- 3 030 332**
**US-A- 3 936 557**
**US-A- 4 090 936**
**US-A- 4 273 889**

(73) Patentinhaber: **CIBA-GEIGY AG, Klybeckstrasse 141, CH-4002 Basel(CH)**

(72) Erfinder: **Roth, Martin, Dr., Oberdorf, CH-1711 Giffers(CH)**
Erfinder: **Meier, Kurt, Dr., Ulmenstrasse 11, CH-4123 Allschwil(CH)**

(56) Entgegenhaltungen: (Fortsetzung)
**US-A- 4 407 759**
**US-A- 4 442 197**
**US-A- 4 632 971**

**CHEMICAL ABSTRACTS, Band 103, Nr. 8, 1985, Seite 49, Zusammenfassung Nr. 55104n, Columbus, Ohio, US; & JP-A-60 51 770**
**PATENT ABSTRACTS OF JAPAN, Band 6, Nr. 237 (P-157)[1115], 25. November 1982; & JP-A-57 136 644**
**CHEMICAL ABSTRACTS, Band 70, Nr. 16, 1969, Seite 3, Zusammenfassung Nr. 68780e, Columbus , Ohio, US; M. KATO et al.: "Cationic copolymerization of o-hydroxystyrene with ethyl vinyl ether and etherification of the polymers", & KOGYO GIJUTSUIN SEN'I KOGYO SHIKENSHO KENKYU HOKOKU 1968, (84), 1-6 000**
**CHEMICAL ABSTRACTS, Band 103, Nr. 16, 1985, Seite 601, Zusammenfassung Nr. 132418x, Columbus, Ohio, US; & JP-A-60 71 657 (AGENCY OF INDUSTRIAL SCIENCES AND TECHNOLOGY) 23. April 1985**
**PATENT ABSTRACTS OF JAPAN, Band 9, Nr. 299 (P-408)[2022], 27. November 1985; & JP-A-60 134 234**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 255 989 B1**

ACTORUM AG

## Beschreibung

Die vorliegende Erfindung betrifft eine Zusammensetzung enthaltend im wesentlichen Polyphenole und Epoxidverbindungen oder Vinylether, ein Verfahren zur Herstellung von Reliefstrukturen und die Verwendung besagter Zusammensetzung als wässrig-entwickelbarer Negativ-Resist.

Negativresists auf Epoxidbasis sind bekannt. In der EP-A 153 904 wird ein Verfahren zur Herstellung von Schutzschichten und von Reliefstrukturen beschrieben, wobei ein solcher strahlungsempfindlicher Film verwendet wird. Das System wird mit organischen Lösungsmitteln entwickelt.

Aus der GB-A 2 009 435 sind rein wässig entwickelbare Negativ-Resists bekannt, worin ein wasserlösliches oder quellbares Bindemittel, u.a. Polyvinylalkohol, und ein Acrylmonomeres als polymerisierbare Komponente verwendet werden. Systeme auf Acrylatbasis sind den Epoxidsystemen im allgemeinen hinsichtlich der mechanischen und elektrischen Eigenschaften, der Temperaturstabilität und der Adhäsion auf dem Substrat unterlegen. Ausserdem sind rein wässrig entwickelbare Systeme im Gegensatz zu alkalisch-wässrig oder sauer-wässrig entwickelbaren Systemen relativ feuchtigkeitsempfindlich und ihre Lagerstabilität ist begrenzt.

In der EP-A 124 292 wird eine wässrige Dispersion eines Epoxidharzes und eines Onium-Photoinitiators beschrieben. Die Zusammensetzung wird zur Herstellung von Schutzschichten und von Reliefabbildungen eingesetzt und ist wässrig entwickelbar. Den Dispersionen werden bevorzugt Netzmittel oder Schutzkolloide, beispielsweise Polyvinylalkohol, zugesetzt. Es ist allerdings nicht jedes Epoxidharz geeignet; so muss in einigen Fällen ein reaktiver Verdünner zugesetzt werden, um die Dispergierbarkeit zu gewährleisten.

Aus der US-A 4 273 889 sind hitzehärtbare Zusammensetzungen aus Epoxidharzen und Poly-isopropenylphenol bekannt, die gegebenenfalls zusätzliche Härtungsbeschleuniger aufweisen können. Die konkret offenbarten Zusammensetzungen weisen im allgemeinen einen zu geringen Gehalt an Polyphenol auf und sind nicht alkalisch-wässrig entwickelbar. Die Härtung erfolgt im allgemeinen durcht Umsetzung der phenolischen Hydroxylgruppen mit den Epoxidgruppen. Für den Fall, dass eine zusätzliche Vernetzung über olefinische Doppelbindungen im Polyphenol erwünscht ist, wird die Verwendung von Photoinitiatoren vorgeschlagen.

Aus der JP-A 60-71 657 sind Kombinationen aus vinylethermodifiziertem Phenol-Novolak und Haloniumsalzen bekannt. Die modifizierten Polyphenole besitzen einen geringen Gehalt an freien phenolischen Hydroxylgruppen, so dass die offenbarten Zusammensetzungen nicht alkalisch-wässrig entwickelbar sind.

Es wurde jetzt gefunden, dass man wässrig-alkalisch entwickelbare Filme auf Epoxidharz- oder Vinyletherbasis herstellen kann, indem man als Bindemittel ein Polyphenol oder ein Gemisch von Polyphenolen verwendet.

Die vorliegende Erfindung betrifft einen alkalisch-wässrig entwickelbaren Negativ-Photoresist bestehend im wesentlichen aus

a) mindestens einem festen, filmbildenden Polyphenol, das einen solch hohen Gehalt an phenolischen Hydroxylgruppen aufweist, so dass die Entwicklung oder die Quellung in einer alkalisch-wässrigen Entwicklerlösung gewährleistet ist,
b) mindestens einer Verbindung, die wenigstens zwei Epoxidgruppen oder wenigstens zwei Vinylethergruppen oder wenigstens eine Epoxid- und eine Vinylethergruppe im Molekül aufweist,
c) mindestens einem Oniumsalz mit Anionen schwacher Nucleophilie oder mindestens einem Metallocensalz als kationischem Photoinitiator für die Komponente b) und
d) gegebenenfalls üblichen Zusätzen.

Das Bindemittel a) ist ein festes, filmbildendes Polyphenol, d.h. ein Polymer, das einen bestimmten Gehalt an phenolischen Hydroxylgruppen aufweist. Dieser sollte zumindest so hoch sein, dass die Entwicklung oder zumindest die Quellung in einer wässrig alkalischen Entwicklerlösung gewährleistet ist.

Geeignete alkalisch-wässrig lösliche, filmbildende Bindemittel a) lassen sich im allgemeinen in folgende Gruppen einteilen:

i) Novolake aus mindestens einem Phenol und mindestens einem Aldehyd,
ii) Homo- und Copolymere von Alkenylphenolen und insbesondere
iii) Homo- und Copolymere von N-Hydroxyphenylmaleinimiden.

Bevorzugt werden dabei die Gruppen ii) und iii). Bevorzugte Novolake i) sind Verbindungen, die sich von einem $C_1$-$C_6$Aldehyd, beispielsweise Formaldehyd und Acetaldehyd, und von einem zweikernigen, bevorzugt jedoch einkernigen, gegebenenfalls substituierten Phenol ableiten. Beispiele für bevorzugte Phenole sind Phenol selbst oder mit ein oder zwei $C_1$-$C_9$Alkylgruppen substituierte Phenole, wie beispielsweise o-, m-oder p-Kresol, Xylenol, p-tert.Butylphenol und o-, m-oder p-Nonylphenol, oder mit ein oder zwei Halogenatomen, bevorzugt Chlor oder Brom, substituierte Phenole, beispielsweise p-Chlorphenol, mit einem Phenylkern substituierte Phenole, wie z.B. p-Phenylphenol, oder auch Phenole mit

mehr als einer phenolischen Gruppe, wie z.B. Resorcin, Bis-(4-hydroxyphenyl)-methan oder 2,2-Bis-(4-hydroxyphenyl)propan.

Bevorzugte Homo- oder Copolymere von Alkenylphenolen ii) sind insbesondere die Verbindungen der Formel I

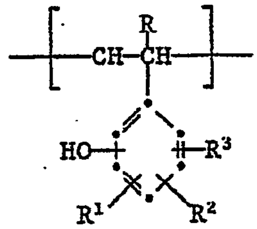

(I),

worin r Wasserstoff oder Methyl ist, $R^1$, $R^2$ und $R^3$ unabhängig voneinander Wasserstoff, $C_1$-$C_4$Alkyl, $C_1$-$C_4$Alkoxy, Halogen, insbesondere Chlor oder Brom, und Methylol bedeuten. Die phenolische Hydroxylgruppe ist in o-, m- oder p-Position zur Alkenylgruppe angeordnet, bevorzugt jedoch in p-Position.

Als Comonomere kommen beispielsweise carboxylgruppenfreie Vinylmonomere in Betracht. Beispiele für solche Monomere sind Styrol, Acryl- und Methacrylsäureester, insbesondere (Meth)acrylsäuremethylester oder (Meth)acrylsäure-2-hydroxyethylester, Acrylamid, Vinylacetat und N-substituierte Maleinimide.

Der Comonomerenanteil der Bindemittel des Typs ii) beträgt bevorzugt 0-50 Mol%, bezogen auf das Gesamtpolymere.

Polymere des Typs ii) sind bekannt und beispielsweise in der DE-OS 2,322,230 und in der EP-A 153,682 beschrieben. Teilweise sind solche Polymere auch im Handel erhältlich.

Bevorzugte Bindemittel des Typs iii) sind Homopolymere von N-Hydroxyphenylmaleinimiden. Dabei handelt es sich bevorzugt um Homopolymere mit der Struktureinheit der Formel II

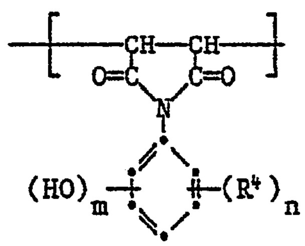

(II),

worin $R^4$ $C_1$-$C_4$Alkyl, $C_1$-$C_4$Alkoxy oder Halogen, insbesondere Chlor oder Brom ist, m 1, 2 oder 3 darstellt, n 0, 1, 2, 3 oder 4 ist und die Summe von m und n höchstens 5 bedeutet, wobei die Reste $R^4$ eines Phenylkernes innerhalb der gegebenen Definitionen unterschiedlich sein können.

Eine weitere bevorzugte Gruppe von Bindemitteln des Typs iii) sind Copolymere auf der Basis von N-Hydroxyphenylmaleinimiden, insbesondere soche mit Strukturelementen der Formel II und carboxylgruppenfreien Vinylverbindungen.

Beispiele für geeignete Vinyl-Comonomere sind:

a) Styroltypen, wie beispielsweise Styrol, α-Methylstyrol, p-Methylstyrol oder p-Hydroxyphenylstyrol;

b) Ester oder Amide α,β-ungesättigter Säuren, wie beispielsweise Acrylsäuremethylester, Acrylsäureamid, die entsprechenden Methacrylverbindungen oder Maleinsäuremethylester;

c) Nitrile von α,β-ungesättigten Säuren, wie beispielsweise Acrylnitril oder Methacrylnitril;

d) halogenhaltige Vinylverbindungen, wie beispielsweise Vinylchlorid, Vinylfluorid, Vinylidenchlorid oder Vinylidenfluorid;

e) Vinylester, wie beispielsweise Vinylacetat oder

f) Vinylether, wie beispielsweise Methylvinylether oder Butylvinylether.

Der Anteil der Vinylkomponente beträgt in der Regel 0 bis 95 Mol%, bevorzugt 0-75 Mol%, besonders bevorzugt 0-50 Mol%, bezogen auf den Gesamtanteil an Monomereinheiten.

Homopolymere aus Struktureinheiten der Formel II und Copolymere mit Vinylverbindungen sind bekannt und werden beispielsweise in der BE-PS 613,801 sowie in Kobunshi Kagaku 26, 593-601 (1969) (Chem. Abstr. 72, 21964n) beschrieben. Ferner berichten Turner et al. in Photopolymer Conference, Ellenville Oct. 1985, S. 35-47 über den Einsatz von solchen Comonomeren mit Vinylverbindungen als Bindemittel in positiven Photolacken.

Eine weitere besonders bevorzugte Gruppe von Bindemitteln des Typs iii) sind Copolymere auf der

Basis von N-Hydroxymaleinimiden, insbesindere solche mit Struckturelementen der Formel II, Allylverbindungen und gegebenenfalls weiteren Vinylverbindungen.

Bei diesen Verbindungen handelt es sich insbesondere um Copolymere enthaltend 30-100 Mol%, bezogen auf das Gesamtpolymer, an Struktureinheiten der Formel II, wie oben definiert, und der Formel III, wobei der Anteil der Reste der Formel II, bezogen auf den gesamten Anteil von II und III, 5 bis 95 Mol% ausmacht

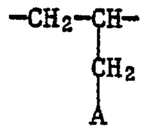 (III),

worin A ausgewählt wird aus der Gruppe der Reste bestehend aus Halogen, Cyano oder Struktureinheiten der Formeln IV bis IX

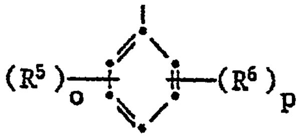 (IV), 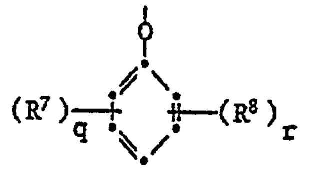 (V),

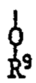 (VI), 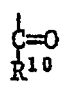 (VII), 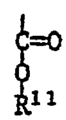 (VIII), 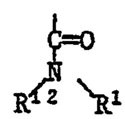 (IX),

worin $R^5$ und $R^7$ unabhängig voneinander Hydroxy- oder Glycidylgruppen der Formel Xa oder Xb darstellen

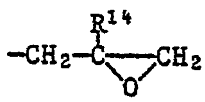 (Xa), 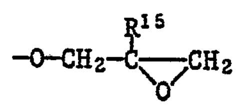 (Xb),

$R^6$ und $R^8$ unabhängig voneinander $C_1$-$C_4$Alkyl, $C_1$-$C_4$Alkoxy oder Halogen sind,
o und q unabhängig voneinander 0, 1, 2 oder 3 sind,
p und r unabhängig voneinander 0, 1, 2, 3, 4 oder 5 bedeuten, wobei die Summen o+p und q+r höchstens 5 betragen dürfen,
$R^9$ Wasserstoff, $C_1$-$C_{20}$Alkyl, ein Glycidylrest der Formel Xa oder ein Rest der Formel VII ist,
$R^{10}$ Wasserstoff, $C_1$-$C_{20}$Alkyl, Cycloalkyl mit 5 bis 7 Ring C-Atomen, Phenyl, Naphthyl oder Benzyl darstellt,
$R^{11}$ Wasserstoff, $C_1$-$C_{20}$Alkyl oder ein Glycidylrest der Formel Xa ist und die Gruppen $R^{12}$ und $R^{13}$ unabhängig voneinander Wasserstoff, $C_1$-$C_{20}$Alkyl, Cycloalkyl mit 5 bis 7 Ring C-Atomen, gegebenenfalls substituiertes Aryl oder Aralkyl oder ein Glycidylrest der Formel Xa sind oder zusammen mit dem gemeinsamen Stickstoffatom einen fünf- oder sechsgliedrigen heterocyclischen Ring bilden und $R^{14}$ und $R^{15}$ unabhängig voneinander Wasserstoff oder Methyl sind, wobei die Reste $R^5$ bis $R^{15}$ und A eines Polymermoleküls innerhalb der gegebenen Definitionen unterschiedlich sein können.

Besonders bevorzugte Bindemittel des Typs iii) sind Copolymere enthaltend 50-100 Mol%, bezogen auf das Gesamtpolymere, an Struktureinheiten der Formeln II und III.

Insbesondere bevorzugt werden Bindemittel des Typs iii) bestehend im wesentlichen aus Strukturelementen der Formeln II und III, worin der Anteil der Elemente II 10-80 Mol%, bezogen auf den Anteil von II und III, ausmacht.

Ganz besonders bevorzugte Bindemittel des Typs iii) sind Copolymere enthaltend Struktureinheiten der Formeln II und III, wie oben definiert, worin A ausgewählt wird aus der Gruppe der Reste der Formeln IV, V, VI, VIII und IX, $R^5$ und $R^7$ Glycidylgruppen der Formeln Xa oder Xb darstellen, $R^9$, $R^{11}$ und mindestens einer der Reste $R^{12}$ oder $R^{13}$ eine Glycidylgruppe der Formel Xa ist und o und q unabängig voneinander 1, 2 oder 3 sind.

Insbesondere bevorzugt werden Bindemittel des Typs iii), die Struktureinheiten der Formeln II und III, wie oben definiert, enthalten, worin A eine Gruppe der Formel VI ist und $R^9$ eine Gruppe der Formel Xa bedeutet, oder worin A eine Gruppe der Formeln IV oder V ist, $R^5$ und $R^7$ Glycidylgruppen der Formel Xb sind, o und q 1 oder 2 bedeuten und p und r 0 sind.

Weitere Bindemittel des Typs iii) sind Copolymere enthaltend mindestens 50 Mol%, bezogen auf das Gesamtpolymere, an Strukturelementen der Formel II und III und als restlichen Anteil Strukturelemente, die sich von carboxylgruppenfreien Vinylverbindungen ausgewählt aus der Gruppe bestehend aus Styroltypen, Estern oder Amiden α,β-ungesättigter Säuren, Nitrilen von α,β-ungesättigten Säuren, halogenhaltigen Vinylverbindungen, Vinylestern und Vinylethern ableiten.

Copolymere aus N-Hydroxyphenylmaleinimiden und Allylverbindungen wurden bislang nicht beschrieben.

Sie lassen sich herstellen, indem man

a) Verbindungen der Formel IIa und IIIa

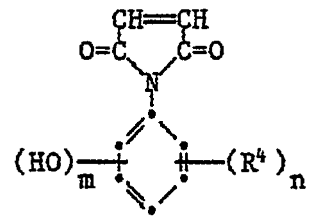 (IIa), 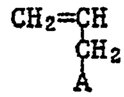 (IIIa),

worin $R^4$, A, m und n die weiter oben definierte Bedeutung besitzen, gegebenenfalls in Anwesenheit weiterer radikalisch polymerisierbarer Monomerer radikalisch polymerisiert oder

b) Verbindungen der Formel IIb und IIIa

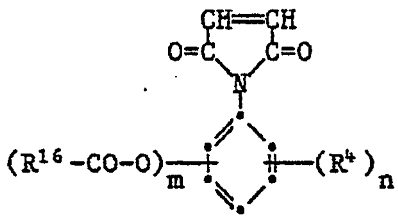 (IIb), 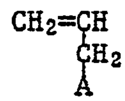 (IIIa),

worin $R^4$, A, m und n die schon weiter oben definierte Bedeutung besitzen und $R^{16}$ ein einwertiger Kohlenwasserstoffrest, bevorzugt $C_1$-$C_4$Alkyl, ist gegebenenfalls in Anwesenheit weiterer radikalisch polymerisierbarer Monomerer radikalisch polymerisiert und das Produkt durch Hydrolyse der $R^3$-CO-Gruppe in ein Copolymeres gemäss der weiter oben gegebenen Definition überführt.

Die Molekulargewichte der N-(Hydroxyphenyl)-maleinimid / Allyl-Copolymeren liegen in der Regel zwischen 500-100000 (Gewichtsmittel).

Die Verbindungen der Formeln IIa und IIb sind an sich bekannt oder können nach Standardreaktionen hergestellt werden. Nähere Einzelheiten dazu sind in der US-PS 4,289,699 zu finden. Die Allylverbindungen der Formel IIIa sind ebenfalls bekannt und teilweise im Handel erhältlich.

Bedeuten $R^1$, $R^2$, $R^3$, $R^4$, $R^6$ oder $R^8$ $C_1$-$C_4$Alkyl, so handelt es sich dabei um geradkettige oder verzweigte, bevorzugt um geradkettige, Alkylreste. Beispiele für solche Gruppen sind Methyl, Ethyl, n-Propyl, Isopropyl, n-Butyl oder sek.Butyl. Bevorzugt wird Methyl.

Als $C_1$-$C_4$Alkoxy besitzt der Alkylteil von $R^1$, $R^2$, $R^3$, $R^4$, $R^6$ oder $R^8$ die oben beispielhaft erwähnten Bedeutungen. Bevorzugt wird Methoxy.

Als Halogen sind $R^1$, $R^2$, $R^3$, $R^4$, $R^6$ oder $R^8$ Fluor, Chlor, Brom oder Iod. Bevorzugt wird Chlor oder Brom, insbesondere Brom.

Sind irgendwelche Reste $C_1$-$C_{20}$Alkyl, so handelt es sich dabei bevorzugt um geradkettige Gruppen. Es können aber auch verzweigte Alkylgruppen sein.

Beispiele für $C_1$-$C_{20}$Alkylreste sind Methyl, Ethyl, n-Propyl, Isopropyl, n-Butyl, sek.Butyl, tert.Butyl, n-Pentyl, Isopentyl, n-Hexyl, n-Heptyl, n-Octyl, 2-Ethylhexyl, n-Nonyl, n-Decyl, n-Undecyl, n-Dodecyl, n-Tetradecyl, n-Hexadecyl, n-Octadecyl oder n-Eicosyl. Bevorzugt werden $C_1$-$C_8$Alkylreste dieses Typs, insbesondere die geradkettigen und ganz besonders Methyl oder Ethyl.

A als Halogen ist Fluor, Chlor, Brom oder Iod. Bevorzugt wird Chlor oder Brom, insbesondere Chlor.

Bei $R^{10}$, $R^{12}$ und $R^{13}$ als Cycloalkyl mit 5 bis 7 Ring C-Atomen handelt es sich inder Regel um Cyclopentyl, Cyclohexyl oder Cycloheptyl, insbesondere jedoch um Cyclohexyl. Diese Reste sind gegebenenfalls alkylsubstituiert.

Als gegebenenfalls substituiertes Aryl sind $R^{12}$ oder $R^{13}$ beispielsweise Phenyl oder Naphthyl. Als Substituenten kommen beispielsweise Alkylgruppen, insbesondere Methyl, Alkoxygruppen, insbesondere Methoxy, Halogenatome, insbesondere Chlor oder Brom, oder Cyanogruppen in Frage.

Spezifische Beispiele für substituierte Arylreste sind o-, m- oder p-Tolyl, Xylyl oder Chlorphenyl. Bevorzugter Arylrest ist Phenyl.

Als gegebenenfalls substituiertes Aralkyl sind $R^{12}$ oder $R^{13}$ beispielsweise Benzyl, α-Methylbenzyl oder α,α-Dimethylbenzyl. Auch hier kommen als Substituenten die oben für Aryl beispielhaft erwähnten Reste in Frage.

Spezifische Beispiele für substituierte Aralkylreste sind 4-Methylbenzyl oder 4-Methoxybenzyl. Bevorzugt wird Benzyl.

Der Index m im Strukurelement der Formel II ist vorzugsweise 1 oder 2, besonders bevorzugt 1. Der Index n im Strukturelement der Formel II ist vorzugsweise 0, 1 oder 2, besonders bevorzugt 0.

Die Komponente b) der erfindungsgemässen Negativ-Resists enthält mindestens eine Verbindung mit wenigstens zwei Epoxidgruppen oder wenigstens zwei Vinylethergruppen oder wenigstens einer Epoxid- und einer Vinylethergruppe im Molekül.

Als Verbindungen mit wenigstens zwei Vinylethergruppen lassen sich beispielsweise Divinylether von aliphatischen, cycloaliphatischen, aromatischen oder araliphatischen Diolen einsetzen.

Beispiele für bevorzugte Verbindungen dieses Typs sind Divinylether von $C_1$-$C_{12}$aliphatischen Diolen, Polyethylenglykolen, Polypropylenglykolen, Polybutylenglykolen, Dimethylolcyclohexanen, von ein- oder zweikernigen aromatischen Diphenolen sowie von einkernigen araliphatischen Diolen.

Beispiele für spezifische Verbindungen dieser Verbindungsklassen sind die Divinylether von Ethylenglykol, Trimethylen-1,3-diol, Tetramethylen-1,4-diol, 1-Methylpropan-1,3-diol, Octamethylen-1,8-diol, Diethylenglykol, Triethylenglycol, Dipropylenglykol, Tripropylenglykol, Dibutylenglykol, Tributylenglykol, $HO-(CH_2)_2-O-(CH_2)_4-O-(CH_2)_2-OH$, Bis-1,4-methylolcyclohexan, Hydrochinon, Resorcin, Bisphenol A, Bisphenol F und Bis-methylolbenzol.

Es lassen sich auch Polyvinylether von Novolaken einsetzen. Bevorzugte Komponenten b) sind Verbindungen mit wenigstens zwei Epoxidgruppen im Molekül.

Daneben können auch kleinere Anteile an monofunktionellen Epoxidharzen in Kombination mit polyfunktionellen Epoxidharzen eingesetzt werden.

Als Komponente b) eignen sich grundsätzlich alle in der Technik der Epoxidverbindungen eingesetzten nicht basischen Harze.

Beispiele für Epoxidharze sind:

I) Polyglycidyl- und Poly-(β-methylglycidyl)-ester erhältlich durch Umsetzung einer Verbindung mit mindestens zwei Carboxylgruppen im Molekül und Epichlorhydrin bzw. Glyzerindichlorhydrin bzw. β-Methyl-epichlorhydrin. Die Umsetzung erfolgt zweckmässig in der Gegenwart von Basen.

Als Verbindung mit mindestens zwei Carboxylgruppen im Molekül können aliphatische Polycarbonsäuren verwendet werden. Beispiele für diese Polycarbonsäuren sind Oxalsäure, Bernsteinsäure, Glutarsäure, Adipinsäure, Pimelinsäure, Korksäure, Azelainsäure, Sebazinsäure oder dimerisierte bzw. trimerisierte Linolsäure.

Es können aber auch cycloaliphatische Polycarbonsäuren eingesetzt werden, wie beispielsweise Tetrahydrophthalsäure, 4-Methyltetrahydrophthalsäure, Hexahydrophthalsäure oder 4-Methylhexahydrophthalsäure.

Weiterhin können aromatische Polycarbonsäuren Verwendung finden, wie beispielsweise Phthalsäure, Isophthalsäure oder Terephthalsäure.

II) Polyglycidyl- oder Poly-(β-methylglycidyl)-ether erhältlich durch Umsetzung einer Verbindung mit mindestens zwei freien alkoholischen Hydroxygruppen und/oder phenolischen Hydroxygruppen und einem geeignet substituierten Epichlorhydrin unter alkalischen Bedingungen, oder in Anwesenheit eines sauren Katalysators und anschliessende Alkalibehandlung.

Ether dieses Typs leiten sich beispielsweise ab von acyclischen Alkoholen, wie Ethylenglykol, Diethylenglykol und höheren Poly-(oxyethylen)-glykolen, Propan-1,2-diol oder Poly-(oxypropylen)-glykolen, Propan-1,3-diol, Butan-1,4-diol, Poly-(oxytetramethylen)-glykolen, Pentan-1,5-diol, Hexan-1,6-diol, Hexan-2,4,6-triol, Glycerin, 1,1,1-Trimethylolpropan, Pentaerythrit, Sorbit, sowie von Polyepichlorhydrinen.

Sie leiten sich aber auch beispielsweise ab von cycloaliphatischen Alkoholen wie 1,3- oder 1,4-Dihydroxycyclohexan, Bis-(4-hydroxycyclohexyl)-methan, 2,2-Bis(4-hydroxycyclohexyl)-propan oder 1,1-Bis-(hydroxymethyl)-cyclohex-3-en.

Die Epoxidverbindungen können sich auch von einkernigen Phenolen ableiten, wie beispielsweise von Resorcin oder Hydrochinon; oder sie basieren auf mehrkernigen Phenolen wie beispielsweise auf Bis-(4-hydroxyphenyl)-methan, 4,4'-Dihydroxydiphenyl, Bis-(4-hydroxyphenyl)-sulfon, 1,1,2,2-Tetrakis-(4-hydroxyphenyl)-ethan, 2,2-Bis-(4-hydroxyphenyl)-propan, 2,2-Bis-(3,5-dibrom-4-hydroxyphenyl)-propan sowie auf Novolaken erhältlich durch Kondensation von Aldehyden, wie beispielsweise Formaldehyd, Acetaldehyd, Chloral oder Furfuraldehyd mit Phenolen wie Phenol, oder mit Phenolen, die im Kern mit Chloratomen oder $C_1$-$C_9$Alkylgruppen substituiert sind, wie beispielsweise 4-Chlorphenol, 2-Methylphenol oder 4-tert. Butylphenol oder erhältlich durch Kondensation mit Bisphenolen, so wie oben beschrieben.

III) Beispiele für Poly-(S-glycidyl)-Verbindungen sind Di-S-glycidylderivate, die sich von Dithiolen, wie beispielsweise Ethan-1,2-dithiol oder Bis-(4-merkaptomethylphenyl)-ether ableiten.

IV) Beispiele für cycloaliphatische Epoxidharze sind Bis-(2,3-epoxicyclopentyl)-ether, 2,3-Epoxicyclopentylglycidylether, 1,2-Bis-(2,3-epoxicyclopentyloxy)-ethan, Vinylcyclohexandioxid, Limonendioxid,

Dicyclopentadiendioxid, 4-Oxatetracyclo[6,2,1,$0^{2,7}$,$0^{3,5}$]undec-9-yl-glycidylether, 1,2-Bis-(4-oxatetracyclo[6,2,1,$0^{2,7}$,$0^{3,5}$]undec-9-yl-oxy)ethan, der 3,4-Epoxicyclohexylmethylester der 3′, 4′-Epoxicyclohexansäure sowie dessen 6,6′-Dimethylderivat, der Bis-(3,4-epoxicyclohexansäureester) des Ethylenglykols oder 3-(3,4-Epoxicyclohexyl)-8,9-epoxi-2,4-dioxaspiro-[5,5]undecan.

Es lassen sich aber auch Epoxidharze verwenden, bei denen die 1,2-Epoxidgruppen an unterschiedliche Heteroatome bzw. funktionelle Gruppen, gebunden sind; zu diesen Verbindungen zählen beispielsweise der Glycidylether-glycidylester der Salicylsäure.

Falls gewünscht, kann eine Mischung von Epoxidharzen in den erfindungsgemässen Zusammensetzungen verwendet werden.

Besonders bevorzugt als Epoxidharze werden Polyglycidylether von Novolaken, die durch Umsetzung von Formaldehyd mit einem Phenol gebildet werden, oder von den oben erwähnten aliphatischen Diolen, insbesondere von Butan-1,4-diol. Ganz besonders bevorzugt als Epoxidharze werden die cycloaliphatischen Epoxidharze.

Besonders bevorzugt als Komponente b) werden Polyglycidylverbindungen, die sich in ungehärtetem Zustand in Wasser dispergieren lassen. Dabei handelt es sich in der Regel um relativ niedermolekulare Epoxidverbindungen mit weiteren polaren Gruppen, wie Hydroxyl-, Ether- oder Estergruppen, welche in einem solchen Ausmass neben den Epoxidgruppen vorhanden sein müssen, so dass die Dispergierbarkeit in alkalisch-wässrigen Lösungen gewährleistet ist. Es kann sich aber auch um relativ hochmolekulare Verbindungen handeln, die neben den Epoxidgruppen einen zur Erzielung der Wasserdispergierbarkeit ausreichenden Anteil an polaren Gruppen besitzen.

Beispiele für bevorzugte niedermolekulare Polyepoxide dieses Typs sind Polyglycidylether, die sich von Tetramethyloltetrahydropyran-4-on oder -4-ol ableiten, sowie Polyglycidylester, die sich von epoxidierten Fettsäuren und Tetramethylolcyclohexanol, -cyclohexanon, -tetrahydropyran-4-ol oder -tetrahydropyran-4-on ableiten. Beispiele für solche Verbindungen sind in der CH-A-358,930 und in der US-A-3,558,535 beschrieben.

Besonders bevorzugte wasserdispergierbare Epoxidharze sind Polyglycidylether von Tetramethylolcyclohexanol oder -cyclohexanon sowie Di- oder Triglycidylglycerinether. Die Tetramethylolcyclohexanol- oder -cyclohexanonglycidylether sind aus der EP-A-135,477 bekannt.

Als Komponente c) der erfindungsgemässen Gemische lässt sich eine Vielzahl bekannter und in der Technik erprobter kationischer Photoinitiatoren für Epoxidharze einsetzen. Dabei handelt es sich beispielsweise um Oniumsalze mit Anionen schwacher Nucleophilie. Beispiele dafür sind Haloniumsalze, wie unter Formel II in der EP-A 153,904 erwähnt, Iodosylsalze, wie unter Formel IV der EP-A 153,904 erwähnt, Sulfoniumsalze, wie unter Formel III der EP-A 153,904 erwähnt, Sulfoxoniumsalze, wie beispielsweise in den EP-A 35,969, 44,274, 54,509 und 164,314 beschrieben oder Diazoniumsalze, wie beispielsweise in der US-A-3,708,296 beschrieben.

Eine Uebersicht über weitere gängige Oniumsalzinitiatoren bietet "UV-Curing, Science and Technology" (Editor: S.P. Pappas, Technology Marketing Corp., 642 Westover Road, Standford, Conneticut, USA).

Bevorzugte Photoinitiatoren sind Metallocensalze, wie beispielsweise in der EP-A 94,914 und ganz besonders in der EP-A 94,915 beschrieben.

Die Ausführungen in diesen Publikationen sind ebenfalls Gegenstand der vorliegenden Beschreibung.

Zu den bevorzugten Photoinitiatoren zählen die Verbindungen der Formeln XI, XII und XIII

$$\left[ R^{16}_{h} - I - R^{17}_{j} \right]^{\oplus} \left[ LQ_{m} \right]^{\ominus} \quad (XI),$$

$$\left[ R^{18} - \overset{\overset{O}{\|}}{I} - R^{19} \right]^{\oplus} \left[ LQ_{m} \right]^{\ominus} \quad (XII),$$

$$\left[ R^{20} - \underset{R^{21}}{\underset{|}{S}} - R^{22} \right]^{\oplus} \left[ LQ_{m} \right]^{\ominus} \quad (XIII),$$

worin h = 1 und j = 1 ist oder h = 2 und j = 0 ist, und $R^{16}$ und $R^{17}$, im Falle h = 1 und j = 1, unabhängig voneinander einwertige carbocyclisch-aromatische $C_6$-$C_{18}$Reste sind, die gegebenenfalls ein bis drei Substituenten tragen, und $R^{16}$, im Falle h = 2 und j = 0, ein zweiwertiger carbocyclisch-aromatischer $C_{12}$-$C_{18}$Rest ist, der gegebenenfalls ein bis drei Substituenten trägt, $R^{18}$, $R^{19}$, $R^{20}$, $R^{21}$ und $R^{22}$ unabhängig voneinander eine der Bedeutungen von $R^{17}$ annehmen, L ausgewählt wird aus der Gruppe bestehend aus B, P, As und Sb, Q ein Halogenatom ist oder ein Teil der Reste Q in einem Anion $LQ_m^-$ auch Hydroxylgruppen sein können und m eine ganze Zahl ist, die der um Eins vergrößerten Wertigkeit von L entspricht.

Beispiele für einwertige carbocyclisch-aromatische $C_6$-$C_{18}$Reste sind Phenyl, Naphthyl, Anthryl und Phenanthryl. Bevorzugt wird Phenyl. Gegebenenfalls vorliegende Substituenten dieser Reste sind Alkyl, bevorzugt $C_1$-$C_6$Alkyl, wie Methyl, Ethyl, n-Propyl, Isopropyl, n-Butyl, n-Pentyl oder n-Hexyl, Alkoxy, bevorzugt $C_1$-$C_6$Alkoxy, wie Methoxy, Ethoxy, n-Propoxy, n-Butoxy, n-Pentoxy oder n-Hexoxy, Halogen, wie Fluor, Chlor, Brom oder Iod, Aminogruppen, Cyanogruppen oder Nitrogruppen.

Ein Beispiel für einen zweiwertigen carbocyclisch-aromatischen $C_{12}$-$C_{18}$Rest ist Biphenyl-2,2'-diyl.

Beispiele für bevorzugte Halogenatome Q sind Chlor oder insbesondere Fluor. Bevorzugte Anionen $LQ_m^-$ sind $BF_4^-$, $PF_6^-$, $AsF_6^-$, $SbF_6^-$ und $SbF_5(OH)^-$.

Zu den ganz besonders bevorzugten Metalloceninitiatoren im Rahmen dieser Erfindung zählen die Verbindungen der Formel XIV

$$\left[ R^{23}(Fe^{II}R^{24})_s \right]_t^{+s} \left[ X \right]_s^{-t} \qquad (XIV),$$

worin s 1 oder 2 bedeutet, t 1, 2 3, 4 oder 5 ist, X für ein nicht nukleophiles Anion steht, $R^{23}$ ein $\pi$-Aren ist und $R^{24}$ ein Anion eines $\pi$-Arens, bevorzugt Cyclopentadienyl, bedeutet.

Beispiele für $\pi$-Arene $R^{23}$ und Anionen von $\pi$-Arenen $R^{24}$ findet man in der EP-A 94,915.

Beispiele für bevorzugte $\pi$-Arene $R^{23}$ sind Toluol, Xylol, Ethylbenzol, Cumol, Methoxybenzol, Methylnaphthalin, Methoxynaphthalin, Pyren, Perylen, Stilben, Diphenylenoxid und Diphenylensulfid.

Ganz besonders bevorzugt man Cumol, Methylnaphthalin oder Stilben.

Beispiele für nicht nukleophile Anionen $X^-$ sind $FSO_3^-$, Anionen von organischen Sulfonsäuren, von Carbonsäuren oder Anionen $[LQ_m]^-$, wie oben definiert.

Bevorzugte Anionen leiten sich ab von teilfluor- oder perfluoraliphatischen oder teilfluor- oder perfluoraromatischen Carbonsäuren, oder insbesondere von teilfluor- oder perfluoraliphatischen oder von teilfluor- oder perfluoraromatischen organischen Sulfonsäuren oder es handelt sich bevorzugt um Anionen $[LQ_m]^-$.

Beispiele für Anionen X sind $BF_4^-$, $PF_6^-$, $AsF_6^-$, $SbF_6^-$, $SbF_5OH^-$, $CF_3SO_3^-$, $C_2F_5$-$SO_3^-$, n-$C_3F_7SO_3^-$, n-$C_4F_9SO_3^-$, n-$C_6F_{13}SO_3^-$, n-$C_8F_{17}SO_3^-$, $C_6F_5SO_3^-$, Phosphorwolframat ($PO_{40}W_{12}^{3-}$) oder Siliciumwolframat ($SiO_{40}W_{12}^{4-}$).

Bevorzugt werden $PF_6^-$, $AsF_6^-$, $SbF_6^-$, $CF_3SO_3^-$, $C_2F_5SO_3^-$, n-$C_3F_7SO_3^-$, n-$C_4F_9SQ_3^-$, n-$C_6F_{13}SO_3^-$ und n-$C_8F_{17}SO_3^-$.

Man kann auch Kombinationen dieser Metallocensalze mit Oxidationsmitteln einsetzen. Solche Kombinationen sind in der EP-A 126,712 beschrieben.

Zur Erhöhung der Lichtausbeute setzt man, je nach Initiatortyp, vorzugsweise Sensibilisatoren zu.

Beispiele dafür sind polycyclische aromatische Kohlenwasserstoffe oder aromatische Ketoverbindungen. Spezifische Beispiele bevorzugter Sensibilisatoren sind in der EP-A 153,904 erwähnt.

Die erfundungsgemässe Mischung kann noch weitere in der Technik der Negativ-Resists übliche Zusätze d) enthalten. Beispiele für solche Zusätze sind Netzmittel, Verlaufsmittel, Stabilisatoren, Farbstoffe, Pigmente, Füllstoffe, Haftvermittler, Weichmacher und Zusatzharze. Zusatzharze werden im allgemeinen verwendet, um die mechanischen Eigenschaften der Schicht zu modifizieren. Beispiele dafür sind Vinylcopolymere auf Styrol- oder Acrylatbasis, Polyester, Polyamide oder Silikonharze. Diese Zusatzharze werden in der Regel in kleinen Mengen, bevorzugt bis 10 Gew.%, bezogen auf die Gesamtmischung, zugesetzt. Sie müssen mit den übrigen Bestandteilen der Mischung verträglich sein und sollten in organischen Lösungsmitteln löslich sein.

Ferner können den Mischungen noch Katalysatoren, Beschleuniger und Härter für die Epoxidkomponente beigegeben werden. Diese Zusätze bewirken bei einer eventuellen thermischen Nachbehandlung eine Nachhärtung der Epoxidkomponente; gegebenenfalls erfolgt dieser Nachhärtungsschritt nach der Entwicklung des Photoresists.

Die erfindungsgemässen Massen enthalten bevorzugt 10-90 Gew.% des Bindemittels a), 5-80 Gew.% des Epoxidharzes oder Vinylethers b), 0,5-20 Gew.% des Photoinitiators c) und 0-15 Gew.% der Zusatzstoffe d). Dabei ergänzen sich die Anteile der einzelnen Komponenten jeweils zu 100 % und die Mengenangeben sind auf die Gesamtmenge bezogen.

Ganz besonders bevorzugt enthalten die Massen, bezogen auf die Gesamtmenge, 30-85 Gew% Bindemittel a), 10-60 Gew.% Epoxidverbindung oder Vinylether b), 1-10 Gew.% Photoinitiator c) und 0-10 Gew.% Zusatzstoffe d).

Zur Herstellung von Beschichtungen löst man die Komponenten a)-c) und gegebenenfalls d) in einem geeigneten Lösungsmittel. Die Wahl des Lösungsmittels und die Konzentration richtet sich hauptsächlich nach der Art der Komponenten und nach dem Beschichtungsverfahren. Das Lösungsmittel soll inert sein, d.h. es soll mit den Komponenten keine chemische Reaktion eingehen und es soll bei der Trocknung nach dem Beschichten wieder entfernt werden können.

Bekannte Beschichtungsverfahren sind z.B.: Schleudern, Tauchen, Rakelbeschichtung, Vorhanggiessverfahren, Aufpinseln, Sprühen oder Reverse-Rollbeschichtung.

Ferner kann die erfindungsgemässe Zusammensetzung als Dry-film von einem temporären, flexiblen Träger auf ein Substrat übertragen werden. Ein solches Verfahren ist z.B. in der EP-A 153,904 beschrieben.

Die Auftragsmenge (Schichtdicke) und Art des Substrates (Schichtträger) sind abhängig vom gewünschten Applikationsgebiet. Besonders vorteilhaft ist es, dass die erfindungsgemässen Zusammensetzungen in weit variablen Schichtdicken eingesetzt werden können. Dieser Schichtdickenbereich umfasst Werte von ca. 0,5 µm bis mehr als 100 µm.

Mögliche Einsatzgebiete der erfindungsgemässen Zusammensetzungen sind die Verwendung als Photoresists für die Elektronik (Galvanoresist, Aetzresist, Lötstoppresist), die Herstellung von Druckplatten, wie Offsetdruckplatten oder Siebdruckformen, der Einsatz beim Formteilätzen oder der Einsatz als Mikroresist bei der Herstellung integrierter Schaltkreise. Dementsprechend unterschiedlich sind die möglichen Schichtträger und die Verarbeitungsbedingungen der beschichteten Substrate.

Für photographische Informationsaufzeichnung dienen z.B. Folien aus Polyester, Celluloseacetat oder mit Kunststoff beschichtete Papiere; für Offsetdruckformen speziell behandeltes Aluminium und für die Herstellung gedruckter Schaltungen kupferkaschierte Laminate. Die Schichtdicken für photographische Materialien und Offsetdruckformen betragen bevorzugt ca. 0,5 bis 10 µm; für gedruckte Schaltungen 1 bis ca. 100 µm.

Nach dem Beschichten wird das Lösungsmittel in der Regel durch Trocknen entfernt und es resultiert eine Schicht des Photoresist auf dem Träger.

Die Lichtempfindlichkeit der erfindungsgemässen Zusammensetzungen reicht in der Regel vom UV-Gebiet (ca. 200 nm) bis ca. 600 nm und umspannt somit einen sehr breiten Bereich. Als Lichtquellen kommen daher eine grosse Anzahl der verschiedensten Typen zur Anwendung. Es sind sowohl Punktquellen als auch flächenförmige Strahler (Lampenteppiche) geeignet. Beispiele sind: Kohlelichtbogenlampen, Xenon-Lichtbogenlampen, Quecksilberdampflampen, gegebenenfalls mit Mettal-Halogeniden dotiert (Metall-Halogenlampen), Fluoreszenzlampen, Argonglühlampen, Elektronenblitzlampen und photographische Flutlichtlampen.

Der Abstand zwischen Lampe und erfundungsgemässem Bildmaterial kann je nach Anwendungszweck und Lampentyp bzw. -stärke variieren, z.B. zwischen 2 cm bis 150 cm. Speziell geeignet sind Laserlichtquellen, z.B. Argonionenlaser oder Kryptonionenlaser mit starken Emissionslinien (Ar-Laser) bei 457, 476, 488, 514, 528 nm. Bei dieser Art der Belichtung ist keine Photomaske im Kontakt mit der Photopolymerschicht mehr nötig; der gesteuerte Laser-Strahl schreibt direkt auf die Schicht. Hier ist die hohe Empfindlichkeit der erfindungsgemässen Materialien sehr vorteilhaft, die hohe Schreibgeschwindigkeiten bei relativ niedrigen Intensitäten erlaubt. Nach dieser Methode können gedruckte Schaltungen in der Elektronikindustrie, lithographische Offsetdruckplatten oder Reliefdruckplatten sowie photographische Bildaufzeichnungsmaterialien hergestellt werden.

Nach der bildmässigen Belichtung des Materials und vor der Entwicklung kann es vorteilhaft sein, eine thermische Behandlung für kürzere Zeit durchzuführen. Dabei werden nur die bereits belichteten Teile thermisch nachgehärtet. Diese thermische Behandlung ist besonders empfehlenswert bei den Metallocen-Photoinitiatoren. Sie kann in konventionellen Oefen, beispielsweise in Konvektionsöfen, stattfinden, aber auch mit IR-Strahlung, beispielsweise durch Bestrahlung mit IR-Lasern, oder in Mikrowellengeräten erfolgen. Die angewandten Temperaturen liegen im allgemeinen bei 50-150°C, bevorzugt bei 80-130°C; die Härtungszeit liegt in der Regel zwischen 1 und 15 Minuten.

Nach der Belichtung und gegebenenfalls thermischen Nachbehandlung werden die unbelichteten Stellen des Photolackes in an sich bekannter Weise mit einem Entwickler entfernt.

Besonders bevorzugt als Entwickler werden wässrig alkalische Lösungen, wie sie auch für die Entwicklung von Naphthodiazidchinonschichten eingesetzt werden. Dazu zählen insbesondere Lösungen von Alkalimetallsilikaten, -phosphaten und -hydroxiden und -carbonaten. Diesen Lösungen können gegebenenfalls noch kleinere Mengen an Netzmitteln und/oder organischen Lösungsmitteln zugesetzt sein.

Entwicklung mit organischen Lösungsmitteln ist ebenfalls möglich. In Frage kommen beispielsweise Cyclohexanon, 2-Ethoxyethanol, Aceton, MEK sowie Mischungen dieser Lösungsmittel.

Nach der Belichtung und Entwicklung kann das Reliefbild nochmals einer thermischen Behandlung unterworfen werden (thermisch Nachhärtung 2. Stufe). Dabei reagieren verbliebene Epoxidgruppen bzw. Vinylethergruppen mit den phenolischen Hydroxylgruppen des Binders oder polymerisieren mit sich selbst. Dazu können die in der Epoxidharz- oder Vinyletherchemie üblichen Katalysatoren und Beschleuniger bereits bei der Beschichtung zugesetzt werden (Komponente d), sie dürfen allerdings die Belichtung nicht stören.

Durch diese zusätzliche thermische Netzwerkbildung werden besonders resistente und dauerhafte Reliefbilder erhalten, die sich, wie auch Beschichtungen gehärteter Epoxidharze, durch gute thermomechanische, chemische und elektrische Eigenschaften, insbesondere Stabilität, Haftung und hohen spezifischen Durchgangswiderstand auszeichnen.

Die Erfindung betrifft daher auch ein Verfahren zur Erzeugung von Reliefstrukturen umfassend folgende Arbeitsschritte:

a) Beschichten eines Substrates mit einer strahlungsempfindlichen Zusammensetzung, wie oben definiert,

b) Belichten des beschichteten Substrates mit einem vorbestimmten Muster aktinischer Strahlung, gegebenenfalls
c) thermische Nachbehandlung,
d) Entwicklungsstufe und
e) gegebenenfalls thermische Nachbehandlung des entwickelten Systems.

Der Begriff 'Belichtung mit einem vorbestimmten Muster aktinischer Strahlung' beinhaltet sowohl die Belichtung durch eine Photomaske, die ein vorbestimmtes Muster enthält, beispielsweise ein Diapositiv, sowie die Belichtung durch einen Laserstrahl, der beispielsweise computergesteuert über die Oberfläche des beschichteten Substrates bewegt wird und auf diese Weise ein Bild erzeugt.
Ferner betrifft die Erfindung ein Verfahren zur Herstellung von Schutzschichten umfassend folgende Arbeitsschritte:

a) Beschichten eines Substrates mit einer strahlungsempfindlichen Zusammensetzung, wie oben definiert,
b) Belichten der strahlungsempfindlichen Schicht in ihrer gesamten Fläche und
c) gegebenenfalls thermische Nachbehandlung des belichteten Systems.

Darüber hinaus betrifft die Erfindung die Verwendung der oben definierten Zusammensetzungen zur Herstellung von Schutzschichten und Reliefstrukturen.
Die folgenden Beispiele erläutern die Erfindung näher.

Herstellungsbeispiele

Beispiel 1

Copolymer aus p-Maleinimidylphenol und Butylvinylether:

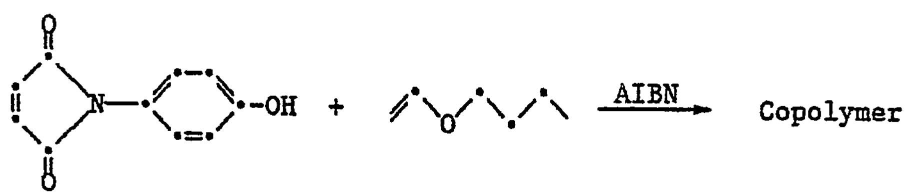


In einen 2, 5 l Sulfierkolben, versehen mit Rührer, Thermometer, Rückflusskühler sowie Stickstoffeinleitung und -ausleitung, gibt man:

378,3 g (2,0 Mol) p-Maleinimidylphenol
220,3 g (2,2 Mol) Butylvinylether
10,3 g (62,7 mMol) α,α'-Azo-bisisobutyronitril
1000,0 g Methylethylketon

Nach dem Inertisieren mit Stickstoff wird die Suspension unter Rühren mit einem Oelbad aufgeheizt. Bei ca. 66° Innentemperatur setzt die Polymerisation unter erheblicher Wärmetönung ein. Das Oelbad wird entfernt und mit einem Eisbad so lange gekühlt, bis der starke Rückfluss des MEK nachlässt. Mit dem Oelbad heizt man dann während 7 Stunden bei einer Temperatur von 75°. Das GPC zeigt dann einen praktisch vollständigen Monomerumsatz.
Für analytische Zwecke wird eine Probe der Polymerlösung in Wasser gefällt und das abfiltrierte Festpolymer im Vakuum (0,01 mbar) mehrere Stunden bei 130°C getrocknet.

$\overline{M_w}$ = 80430 GPC, Polystyrol Standards

$\overline{M_n}$ = 32336 GPC, Polystyrol Standards
Hydroxylgruppengehalt = 4,10 Val/kg (titrimetrisch).

Anwendungsbeispiele

Beispiel I

2 Beschichtungsformulierungen stellt man folgendermassen her:

Resin® M = Poly-p-vinylphenol

| | Ia | Ib |
|---|---|---|
| Resin® M (Maruzen Oil, Japan) ($\bar{M}_w$ ca. 6000) | 12,00 g | - |
| Resin® M (Maruzen Oil, Japan) ($\bar{M}_w$ ca. 20000) | - | 12,00 g |
| 3,4-Epoxicyclohexylmethyl-3,4-epoxicyclohexancarboxylat | 8,00 g | 8,00 g |
| Photoinitiator $\{[C_5H_5]Fe^{II}[Stilben]\}^+PF_6^-$ 10 %-Lösung in Cyclohexanon | 10,00 g | 10,00 g |
| Orasolrot B (Ciba-Geigy) Farbstoff | 0,04 g | 0,04 g |
| Methylethylketon/Methylcellosolve 1:1 | 20,00 g | 20,00 g |

Unter Gelblicht beschichtet man die Lösungen mittels eines Ziehrakel in 100 µm Nassfilmdicke auf eine gereinigte, kupferkaschierte Leiterplatte. Man trocknet 30 Minunten bei 80°C. Die Dicke der lichtempfindlichen Schicht beträgt ca. 40 µm. Belichtet wird durch ein Stouffer Sensitivity guide (Inkremente der opt. Dichte = 0,15) neben einem Stouffer resolution guide. Lichtquelle ist eine 5000 Watt Metall-Halogenlampe (Sylvania M 061) im Abstand von 65 cm zum Vakuumrahmen. Belichtungszeit ist 15 Sekunden. Anschliessend erwärmt man die Platte während 10 Minuten bei 100°C und entwickelt die auf Raumtemperatur abgekühlte Platte im Entwickler (A) bei 20° während 90 Sekunden.

Resultat:

Der kopierte Stufenkeil weist 12-13 Halbtonstufen (Form Ia) oder 13-14 (Form Ib) auf. Das Resistbild kann z.B. als Galvanoresist für die Semi-Additiv-Technik dienen.

Entwickler (A)

75,0 g Natrium-metasilicat-pentahydrat
0,4 g Supronic® B50 (ABM Chemicals Ltd., Stockport Cheshire GB); Netzmittel
925,0 g Deionisiertes Wasser

Beispiel II

Beschichtungslösung:

| | |
|---|---|
| Polymer aus Beispiel 1; 41 %-Lösung in MEK (Binderpolymer) | 58,50 g |
| 3,4-Epoxicyclohexylmethyl-3,4-epoxicyclohexancarboxylat | 16,00 g |
| Photoinitiator $\{[C_5H_5]Fe^{II}[Stilben]\}^+PF_6^-$ 10 %-Lösung in Cyclohexanon | 20,00 g |
| Orasolrot B (Ciba-Geigy) Farbstoff | 0,08 g |
| Methylethylketon/Methylcellosolve 1:1 | 5,50 g |

Diese Lösung beschichtet man wie in Beispiel I beschrieben auf kupferkaschierte Laminate und trocknet zu einer ca. 40 µm dicken Beschichtung. Nach der Belichtung gemäss Beispiel I erwärmt man 10 Minuten bei 120°C und entwickelt die erkaltete Platte mit einem verdünnten Entwickler Ⓐ (A:$H_2O$ = 1:1).

<u>Resultat:</u>

Belichtungszeit: 15 Sekunden
Thermische Behandlung: 10 Minuten/120°
Entwicklungszeit: Entwickler Ⓐ:$H_2O$ = 1:1 v/v 20°C 60 Sekunden
Kopierter Stufenkeil: Letzte abgebildete Stufe = Nr. 14

Das entwickelte Resistbild lässt sich als Aetzmaske zur Wegätzung des Kupfers in den üblichen Bädern (z.B. $FeCl_3$-Lösung) verwenden.

<u>Beispiel III</u>

Hier ist die Herstellung einer wässrig-alkalisch entwickelbaren Negativ-Offsetplatte beschrieben.

Beschichtungslösung:

Die Beschichtungslösung des Beispiels II (40%-ig in MEK/MCS 1:1) wird mit MEK/MCS 1:1 auf 10%-Feststoffgehalt verdünnt. Die Lösung wird mittels Schleuderbeschichtung (30 Sekunden bei 500 Touren/Min.) auf ein elektrolytisch aufgerauhtes und anodisiertes Alumiumblech (Offsetplattensubstrat) aufgetragen und 15 Minuten bei 80° getrocknet. Es resultiert ein Beschichtungsgewicht von 1,3 g/m². Man belichet wie in Beispiel I beschrieben durch einen Stouffer-Testkeil. Lichtquelle ist in diesem Fall eine 2000 Watt Metall-Halogenlampe in Abstand von 75 cm zum Vakuumrahmen. Nach der Belichtung erfolgt die thermische Behandlung und die Entwicklung. Das entwickelte Bild wird, wie in der Lithographie üblich, mit fetter, schwarzer Druckfarbe eingefärbt.

<u>Resultat:</u>

Belichtungszeit: 100 Sekunden
Thermische Behandlung: 10 Min./120°C
Entwicklungszeit: Entwickler Ⓐ:$H_2O$ = 1:1 v/v 20°C 45 Sekunden
Kopierter Stufenkeil: Letzte abgebildete Stufe = Nr. 13

Diese Druckplatte erlaubt eine hohe Auflagenhöhe, dies als Folge der guten mechanischen (Abrieb)eigenschaften der vernetzten Bildteile.

<u>Beispiel IV</u>

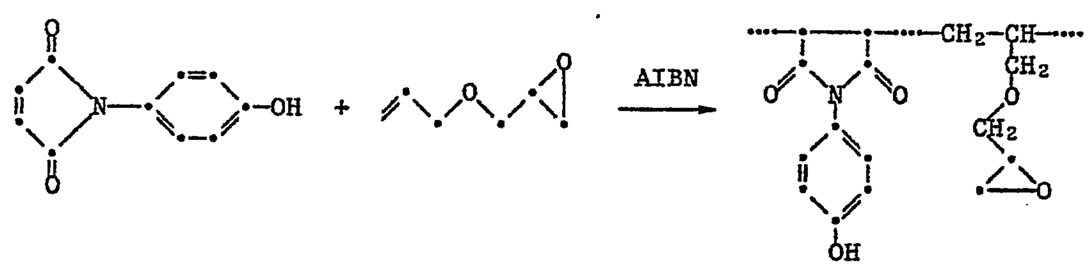


Eine 2,5 l Sulfierkolben, versehen mit Rührer, Thermometer, Rückflusskühler sowie Stickstoffeinleitung und -ausleitung, beschickt man mit:

50,0 g ( 0,264 Mol) p-Maleinimidylphenol
270,6 g ( 2,37 Mol) Allylglycidylether
0,9 g α,α'-Azobisisobutyronitril (AIBN).

Nach dem Inertisieren mit Stickstoff wird die Suspension unter Rühren mit einem Oelbad aufgeheizt. Bei ca. 70°C Innentemperatur setzt die Polymerisation unter erheblicher Wärmetönung ein. Das Oelbad wird entfernt und mit einem Eisbad für ca. 10 Min. gekühlt. Mit dem Oelbad heizt man dann während 7 Stunden bei einer Temperatur von ca. 70°C. Die gelbliche Suspension wird unter gutem Rühren in 4000 ml Toluol eingetragen, die Ausfällung abfiltriert und im Vakuum während 14 Stunden bei 60°C getrocknet.

Man löst das Festprodukt in Aceton und fällt nochmals aus Toluol. Nach dem Trocknen im Vakuum isoliert man das farblose, feste Copolymer.

Elementaranalyse:

gefunden %: C 62,07, H 6,60, N 3,92

$\overline{M}_w$ = 4600 Gel-Permeations Chromatographie (Polystyrol Standards)

$\overline{M}_n$ = 2529 Gel-Permeations Chromatographie (Polystyrol Standards)

Zusammensetzung des statistichen Copolymeren:

p-Maleinimidylphenol/Allylglycidylether = 40,6/59,4 Mol% (aus Elementaranalyse)
Epoxigruppengehalt = 3,7 Val/kg (titrimetrisch)

Beschichtungslösung:

| | |
|---|---|
| Poly(p-maleinimidylphenol-co-allylglycidylether | 1,80 g |
| 3,4-Epoxicyclohexylmethyl-3,4-epoxicyclohexancarboxylat | 1,20 g |
| Photoinitiator $\{[C_5H_5]Fe^{II}[Stilben]\}^+PF_6^-$ 10%-Lösung in Cyclohexanon | 1,50 g |
| Orasolrot B (Ciba-Geigy) Farbstoff | 0,006 g |
| Methylethylketon/Methylcellosolve 1:1 | 5,50 g |

Die Lösung wird analog zu Beispiel I auf ein kupferkaschiertes Laminat beschichtet. Beschichtungsauftrag trocken = ca. 25 g/m². Nach der Belichtung gemäss Beispiel I erwärmt man 10 Minuten bei 120° und entwickelt mit Entwickler (A).

Resultat:

Belichtungszeit: 15 Sekunden
Thermische Behandlung: 10 Min./120°
Entwicklungszeit: Entwickler (A) 20°C 30 Sekunden
Keilkopie: Letzte abgebildete Stufe = Nr. 9

## Beispiel V:

Beschichtungslösung:

| | |
|---|---|
| Resin® M (Maruzen Oil, Japan) ($\overline{M}_w$ ca. 6000) | 2,50 g |
| Bisphenol-A-diglycidylether | 3,00 g |
| ($\eta^6$-Cumol) ($\eta^5$-cyclopentadienyl)eisen(II)-hexafluoroantimonat | 0,16 g |
| Isopropylthioxanthon | 0,08 g |
| Kristallviolett | 0,004 g |
| Cyclohexanon | 8,00 g |

Die Verarbeitung erfolgt analog wie unter Beispiel I beschrieben:

Belichtungszeit: 45 Sekunden;
Härtung : 20 Minuten bei 110°C.
Resultat: Der kopierte Stufenkeil weist 8-9 Halbtonstufen auf.

## Beispiel VI:

Beschichtungslösung:

| | |
|---|---|
| Resin® M (Maruzen Oil, Japan) ($\overline{M}_w$ ca. 6000) | 2,50 g |
| Bisphenol-A-diglycidylether | 3,00 g |
| Triphenylsulfonium-hexafluorophosphat | 0,16 g |
| Isopropylthioxanthon | 0,08 g |
| Kristallviolett | 0,004 g |
| Cyclohexanon | 8,00 g |

Die Verarbeitung erfolgt analog wie unter Beispiel I beschrieben:
Belichtungszeit: 45 Sekunden;
Härtung : 20 Minuten bei 110°C.
Resultat: Der kopierte Stufenkeil weist 2-3 Halbtonstufen auf.

## Beispiel VII:

Beschichtungslösung:

| | |
|---|---|
| Resin® M (Maruzen Oil, Japan) ($\overline{M}_w$ ca. 6000) | 2,50 g |
| Bisphenol-A-diglycidylether | 3,00 g |
| Diphenyliodonium-hexafluoroarsenat | 0,16 g |
| Isopropylthioxanthon | 0,08 g |
| Kristallviolett | 0,004 g |
| Cyclohexanon | 8,00 g |

Die Verarbeitung erfolgt analog wie unter Beispiel I beschrieben:
Belichtungszeit: 45 Sekunden;
Härtung : 20 Minuten bei 60°C.
Resultat: Der kopierte Stufenkeil weist 10-11 Halbtonstufen auf.

**Beispiel VIII:**

**Beschichtungslösung:**

| | |
|---|---|
| Technischer Kresolnovolak (Alnovol PN 430, Hoechst) | 2,50 g |
| Bisphenol-A-diglycidylether | 3,00 g |
| ($\eta^6$-Cumol) ($\eta^5$-cyclopentadienyl)eisen(II)-hexafluoroantimonat | 0,16 g |
| Isopropylthioxanthon | 0,08 g |
| Kristallviolett | 0,004 g |
| Cyclohexanon | 8,00 g |

Die Verarbeitung erfolgt analog wie unter Beispiel I beschrieben:
Belichtungszeit: 45 Sekunden;
Härtung : 20 Minuten bei 100°C.
Resultat: Der kopierte Stufenkeil weist 6-7 Halbtonstufen auf.

**Beispiel IX:**

**Beschichtungslösung:**

| | |
|---|---|
| Resin® M (Maruzen Oil, Japan) ($\bar{M}_w$ ca. 6000) | 2,50 g |
| Bisphenol-A-di(2-vinyloxy-ethyl)-ether | 3,00 g |
| ($\eta^6$-Cumol)($\eta^5$-cyclopentadienyl)eisen(II)-hexafluoroantimonat | 0,016 g |
| Isopropylthioxanthon | 0,08 g |
| Kristallviolett | 0,004 g |
| Cyclohexanon | 8,00 g |

Die Verarbeitung erfolgt analog wie unter Beispiel I beschrieben:
Belichtungszeit: 45 Sekunden;
Härtung : 20 Minuten bei 50°C.
Resultat: Der kopierte Stufenkeil weist 10 Halbtonstufen auf.

Beispiel X:

Beschichtungslösung:

| | |
|---|---|
| Resin® M*) (Maruzen Oil, Japan) | 2,50 g |
| 2,2,6,6-Tetra-(2,3-epoxy-1-propoxy-methyl)-cyclohexanol-1 | 2,50 g |
| ($\eta^6$-Cumol)($\eta^5$-cyclopentadienyl)eisen(II)-hexafluoro-antimonat | 0,16 g |
| Isopropylthioxanthon | 0,08 g |
| Kristallviolett | 0,004 g |
| Cyclohexanon | 8,00 g |

*) Poly-p-vinylphenol; $\bar{M}_w$ ca. 6000

Die Beschichtungslösung wird mit einem 100 µ Drahtrakel auf eine Leiterplatte aufgebracht. Der vorerst nasse Film wird bei 80°C während 30 Minuten getrocknet. Die Belichtung erfolgt mit der in Beispiel I beschriebenen Belichtungsapparatur. Belichtet wird 45 Sekunden durch eine Maske, die die Lötaugen abdeckt. Die belichtete Platte wird 10 Minuten bei 110°C vorgehärtet, anschliessend im Entwickler (A) während 90 Sekunden entwickelt und 1 Stunde bei 135°C nachgehärtet. Die mit Flussmittel (Lösung aus 26 g Rein-Kolophonium in 100 ml Isopropanol) beschichtete Platte wird mit der Beschichtung nach unten auf ein bei 270°C heisses Lötbad (bestehend aus 50 % Blei und 50 % Zinn) gelegt. Nach 10 Sekunden wird die Platte vom Lötbad entfernt, nach Abkühlen durch Waschen mit Isopropanol vom Flussmittel befreit und mit Pressluft getrocknet.

Resultat: Die Beschichtung zeigt keinerlei sichtbare Veränderung wie Risse, Blasen, Poren oder haftende Zinn/Blei-Rückstände.

Beispiel XI:

Beispiel X wird wiederholt mit der Aenderung, dass das Epoxid 2,2,6,6-Tetra-(2,3-epoxy-1-propoxymethyl)-cyclohexanol-1 durch die gleiche Menge an Glycerinpolyglycidylether (Grilonit G 1705, EMS-CHEMIE AG) ersetzt wird.

Resultat: Keine sichtbare Veränderung der Beschichtung nach dem Lötbad (10 Sekunden/270°C)

Beispiel XII:

Beschichtungslösung:

| | |
|---|---|
| Resin®M (Maruzen Oil, Japan: $\bar{M}_w$ ~ 6000) | 125 g |
| Glycerinpolyglycidylether (Grilonit G1705, EMS-CHEMIE AG) | 125 g |
| ($\eta^6$-Cumol)($\eta^5$-cyclopentadienyl)eisen(II)hexafluoro-antimonat | 8 g |
| Isopropylthioxanthon | 4 g |
| Kristallviolett | 0,2 g |
| 1-Methoxy-2-propanol | 150 g |
| 3-Ethoxy-propionsäureethylester | 150 g |

Die Beschichtungslösung wird mit einer elektrostatischen Sprühbeschichtungsanlage mit Hochrotationsglocke (T = 25'000 U/min.) bei einer Feldstärke von 0,3 kV/mm auf eine kupferkaschierte Epoxidplatte appliziert.

Nach dem Trocknen bei 80°C wird eine 50 µm dicke gleichmässige Beschichtung erhalten.

Belichtung durch eine Maske mit Leiterbild (45 Sekunden) Härtung (20 min. bei 110°C) und Entwicklung erfolgt gemäss Beispiel I.

Die Platte wird nach folgendem Arbeitsschema in einem galvanischen Kupferbad behandelt:

- Reinigen durch 1-minütiges Tauchen in eine Lösung von BUZ-R bei 25°C (Dr.Ing. Max Schlötter GmbH + Co.AG.),
- 1 Min mit fliessendem Wasser spülen,
- Anätzen in einer Lösung von 120 g ENPLATE AD485 (IMASA AG, Dällikon, Schweiz) und 20 ml konzentrierter Schwefelsäure pro Liter Wasser,
- Saures Kupferbad AC der Firma Erne AG, Dällikon, 75 Min bei ca. 25°C und einer Stromdichte von 3 $A/dm^2$,
- Spülen mit Leitungswasser.

Nach der galvanischen Behandlung der Platte weist der Resist keine Beschädigungen wie Blasen, Risse etc, auf und ist durch Strippen mit 1N Natronlauge/Isopropanol 10:1 sauber entfernbar. Der erhaltene Kupferüberzug hat eine Dicke von ca. 50 µm.

**Patentansprüche**

1. Alkalisch-wässrig entwickelbare Negativ-Photoresistzusammensetzung bestehend im wesentlichen aus

a) mindestens einem festen, filmbildenden Polyphenol, das einen solch hohen Gehalt an phenolischen Hydroxylgruppen aufweist, so dass die Entwicklung oder die Quellung in einer alkalisch-wässrigen Entwicklerlösung gewährleistet ist,

b) mindestens einer Verbindung, die wenigstens zwei Epoxidgruppen oder wenigstens zwei Vinylethergruppen oder wenigstens eine Epoxid-und eine Vinylethergruppe im Molekül aufweist,

c) mindestens einem Oniumsalz mit Anionen schwacher Nucleophilie oder mindestens einem Metallocensalz als kationischem Photoinitiator für die Komponente b) und gegebenenfalls

d) üblichen Zusätzen.

2. Zusammensetzung gemäss Anspruch 1, worin Komponente b) mindestens eine Verbindung ist, die wenigstens zwei Epoxidgruppen im Molekül aufweist.

3. Zusammensetzung gemäss Anspruch 1, worin das Bindemittel a) ausgewählt wird aus der Gruppe bestehend aus

i) Novolaken aus mindestens einem Phenol und mindestens einem Aldehyd,

ii) Homo- und Copolymeren von Alkenylphenolen und

iii) Homo- und Copolymeren von N-Hydroxyphenylmaleinimiden.

4. Zusammensetzung gemäss Anspruch 3, worin der Novolak i) sich ableitet von einem zweikernigen oder einkernigen gegebenenfalls substituierten Phenol und einem $C_1$-$C_6$Aldehyd oder das Homo- oder Copolymere von Alkenylphenolen ii) das Strukturelement der Formel I enthält

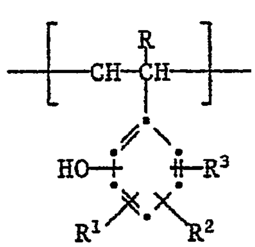

(I),

worin R Wasserstoff oder Methyl ist, $R^1$, $R^2$ und $R^3$ unabhängig voneinander Wasserstoff, $C_1$-$C_4$Alkyl, $C_1$-$C_4$Alkoxy, Halogen und Methylol bedeuten oder das Homo- oder Copolymere von N-Hydroxyphenylmaleinimid iii) das Strukturelement der Formel II enthält

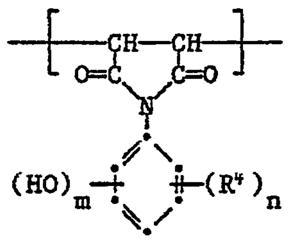

(II),

worin $R^4$ $C_1$-$C_4$Alkyl, $C_1$-$C_4$Alkoxy oder Halogen ist, m 1, 2 oder 3 darstellt, n 0, 1, 2, 3 oder 4 ist und die Summen von m und n höchstens 5 bedeutet, wobei die Reste $R^4$ eines Phenylkernes innerhalb der gegebenen Definitionen unterschiedlich sein können.

5. Zusammensetzung gemäss Anspruch 4, worin das Bindemittel iii) ein Copolymeres auf Basis von Alkenylphenolen und carboxylgruppenfreien Vinylverbindungen ist oder
das Bindemittel iii) ein Copolymeres auf Basis von N-Hydroxyphenylmaleinimid und carboxylgruppenfreien Vinylverbindungen ist.

6. Zusammensetzung gemäss Anspruch 4 enthaltend 30-100 Mol%, bezogen auf das Gesamtpolymere, an Struktureinheiten der Formel II und der Formel III, wobei der Anteil der Reste der Formel II, bezogen auf den gesamten Anteil von II und III, 5 bis 95 Mol% ausmacht

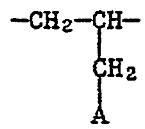

(III),

worin A ausgewählt wird aus der Gruppe der Reste bestehend aus Halogen, Cyano oder Struktureinheiten der Formeln IV bis IX

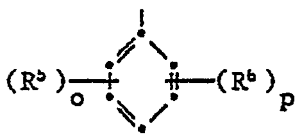 (IV), 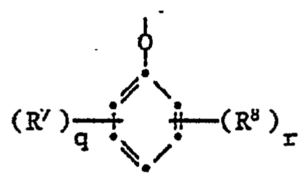 (V),

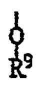 (VI), 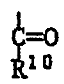 (VII), 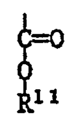 (VIII), 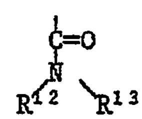 (IX),

worin $R^5$ und $R^7$ unabhängig voneinander Hydroxy- oder Glycidylgruppen der Formel Xa oder Xb darstellen

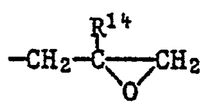 (Xa), 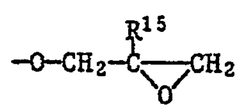 (Xb),

$R^6$ und $R^8$ unabhängig voneinander $C_1$-$C_4$Alkoxy oder Halogen sind,
o und q unabhängig voneinander 0, 1, 2 oder 3 sind,
p und r unabhängig voneinander 0, 1, 2, 3, 4 oder 5 bedeuten, wobei die Summen o+p und q+r höchstens 5 betragen dürfen,
$R^9$ Wasserstoff, $C_1$-$C_{20}$Alkyl, ein Glycidylrest der Formel Xa oder ein Rest der Formel VII ist,
$R^{10}$ Wasserstoff, $C_1$-$C_{20}$Alkyl, Cycloalkyl mit 5 bis 7 Ring C-Atomen, Phenyl, Naphthyl oder Benzyl darstellt,
$R^{11}$ Wasserstoff, $C_1$-$C_{20}$Alkyl oder ein Glycidylrest der Formel Xa ist und die Gruppen $R^{12}$ und $R^{13}$ unabhängig voneinander Wasserstoff, $C_1$-$C_{20}$Alkyl, Cycloalkyl mit 5 bis 7 Ring C-Atomen, gegebenenfalls

substituiertes Aryl oder Aralkyl oder ein Glycidylrest der Formel Xa sind oder zusammen mit dem gemeinsamen Stickstoffatom einen fünf- oder sechsgliedrigen heterocyclischen Ring bilden und $R^{14}$ und $R^{15}$ unabhängig voneinander Wasserstoff oder Methyl sind, wobei die Reste $R^5$ bis $R^{15}$ und A eines Polymermoleküls innerhalb der gegebenen Definitionen unterschiedlich sein können.

7. Zusammensetzung gemäss Anspruch 6 enthaltend Copolymere mit 50-100 Mol%, bezogen auf das Gesamtpolymere, an Struktureinheiten der Formeln II und III.

8. Zusammensetzungen gemäss Anspruch 6, worin das Bindemittel im wesentlichen aus Strukturelementen der Formeln II und III besteht, worin der Anteil der Elemente II 10-80 Mol%, bezogen auf den Anteil von II und III, ausmacht.

9. Zusammensetzungen gemäss Anspruch 6, worin A ausgewählt wird aus der Gruppe der Reste der Formeln IV, V, VI, VIII und IX, $R^5$ und $R^7$ Glycidylgruppen der Formeln Xa oder Xb darstellen, $R^9$, $R^{11}$ und mindestens einer der Reste $R^{12}$ oder $R^{13}$ eine Glycidylgruppe der Formel Xa ist und o und q unabängig voneinander 1, 2 oder 3 sind.

10. Zusammensetzungen gemäss Anspruch 6, worin A eine Gruppe der Formel VI ist und $R^9$ eine Gruppe der Formel Xa bedeutet, oder worin A eine Gruppe der Formeln IV oder V ist, $R^5$ und $R^7$ Glycidylgruppen der Formeln Xb sind, o und q 1 oder 2 bedeuten und p und r 0 sind.

11. Zusammensetzungen gemäss Anspruch 6 enthaltend Copolymere mit mindestens 50 Mol%, bezogen auf das Gesamtpolymere, an Strukturelementen der Formeln II und III und als restlichen Anteil Strukturelemente, die sich von carboxylfreien Vinylverbindungen ausgewählt aus der Gruppe bestehend aus Styroltypen, Estern oder Amiden $\alpha,\beta$-ungesättigter Säuren, Nitrilen von $\alpha,\beta$-ungesättigten Säuren, halogenhaltigen Vinylverbindungen, Vinylestern und Vinylethern ableiten.

12. Zusammensetzungen gemäss Anspruch 1, worin die Komponente b) sich ableitet von
einem Polyglycidylether von Novolaken, die durch Umsetzung von Formaldehyd mit einem Phenol gebildet werden, Diglycidylethern von aliphatischen Diolen und cycloaliphatischen Epoxidharzen.

13. Zusammensetzungen gemäss Anspruch 1, worin Komponente b) eine Polyglycidylverbindung ist, die sich in ungehärtetem Zustand in Wasser dispergieren lässt.

14. Zusammensetzungen gemäss Anspruch 13, worin Komponente b) ein Polyglycidylether von Tetramethylolcyclohexanol oder -cyclohexanon oder ein Di- oder Triglycidylglycerinether ist.

15. Zusammensetzungen gemäss Anspruch 1, worin der Photoinitiator c) ausgewählt wird aus den Verbindungen der Formeln XI, XII und XIII

$$\left[ R^{16}_{h}-I-R^{17}_{j} \right]^{\oplus} \left[ LQ_m \right]^{\ominus} \quad (XI),$$

$$\left[ R^{18}-\overset{\overset{O}{\parallel}}{I}-R^{19} \right]^{\oplus} \left[ LQ_m \right]^{\ominus} \quad (XII),$$

$$\left[ R^{20}-\underset{\underset{R^{21}}{|}}{S}-R^{22} \right]^{\oplus} \left[ LQ_m \right]^{\ominus} \quad (XIII),$$

worin h = 1 und j = 1 ist oder h = 2 und j = 0 ist, und $R^{16}$ und $R^{17}$, im Falle h = 1 und j = 1, unabhängig voneinander einwertige carbocyclischaromatische $C_6$-$C_{18}$Reste sind, die gegebenenfalls ein bis drei Substituenten tragen, und $R^{16}$, im Falle h = 2 und j = 0, ein zweiwertiger carbocyclisch-aromatischer $C_{12}$–$C_{18}$Rest ist, der gegebenenfalls ein bis drei Substituenten trägt, $R^{18}$, $R^{19}$, $R^{20}$, $R^{21}$ und $R^{22}$ unabhängig voneinander eine der Bedeutungen von $R^{17}$ annehmen, L ausgewählt wird aus der Gruppe bestehend aus B, P, As und Sb, Q ein Halogenatom ist oder ein Teil der Reste Q in einem Anion $LQ_m^-$ auch Hydroxylgruppen sein können und m eine ganze Zahl ist, die der um Eins vergrösserten Wertigkeit von L entspricht.

16. Zusammensetzungen gemäss Anspruch 1, worin der Photoinitiator c) eine Verbindung der Formel XIV ist

$$\left[ R^{23}(Fe^{II}R^{24})_s \right]^{+s}_{t} \left[ X \right]^{-t}_{s} \quad (XIV),$$

worin s1 oder 2 bedeutet, t1, 2, 3, 4 oder 5 ist, X für ein nicht nukleophiles Anion, insbesondere für $PF_6^-$, $AsF_6^-$, $SbF_6^-$, $CF_3SO_3^-$, $C_2F_5SO_3^-$, n-$C_3F_7SO_3^-$, n-$C_4F_9SO_3^-$, n-$C_6F_{13}SO_3^-$ und n-$C_8F_{17}SO_3^-$

steht, $R_{23}$ ein π-Aren ist und $R_{24}$ ein Anion eines π-Arens, insbesondere ein Cyclopentadienylanion, bedeutet.

17. Verfahren zur Erzeugung von Reliefstrukturen umfassend folgende Arbeitsschritte:
a) Beschichten eines Substrates mit einer strahlungsempfindlichen Zusammensetzung gemäss Anspruch 1,
b) Belichten des beschichteten Substrates mit einem vorbestimmten Muster aktinischer Strahlung, gegebenenfalls
c) thermische Nachbehandlung,
d) Entwicklungsstufe und gegebenenfalls
e) thermische Nachbehandlung des entwickelten Systems.

18. Verfahren zur Erzeugung von Schutzschichten umfassend folgende Arbeitsschritte:
a) Beschichten eines Substrates mit einer strahlungsempfindlichen Zusmmensetzung gemäss Anspruch 1,
b) Belichten der strahlungsempfindlichen Schicht in ihrer gesamten Fläche und
c) gegebenenfalls thermische Nachbehandlung.

19. Verwendung der Zusammensetzung gemäss Anspruch 1 zur Herstellung von Schutzschichten und Reliefstrukturen.

**Claims**

1. A negative photoresist composition which can be developed under alkaline-aqueous conditions consisting essentially of
a) at least one solid, film-forming polyphenol which has a content of phenolic hydroxyl groups which is high enough to guarantee development or swelling in an alkaline-aqueous developer solution,
b) at least one compound which contains at least two epoxide groups or at least two vinyl ether groups or at least one epoxide and one vinyl ether group in the molecule,
c) at least one onium salt having weakly nucleophilic anions or at least one metallocene salt as cationic photoinitiator for component b) and
d) if appropriate customary additives.

2. A composition according to claim 1, in which component b) is at least one compound which contains at least two epoxide groups in the molecule.

3. A composition according to claim 1, in which binder a) is selected from the group consisting of
i) novolaks of at least one phenol and at least one aldehyde,
ii) homo- and copolymers of alkenylphenols and
iii) homo- and copolymers of N-hydroxyphenylmaleimides.

4. A composition according to claim 3, in which the novolak i) is derived from a binuclear or mononuclear substituted or unsubstituted phenol and a $C_1$–$C_6$aldehyde, or the homo- or copolymer of an alkenylphenol ii) contains the structural element of the formula I

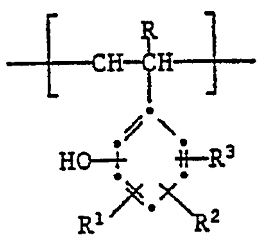

(I),

in which R is hydrogen or methyl and $R^1$, $R^2$ and $R^3$ independently of one another are hydrogen, $C_1$-$C_4$alkyl, $C_1$-$C_4$alkoxy, halogen or methylol, or the homo- or copolymer of N-hydroxyphenylmaleimide iii) contains the structural element of the formula II

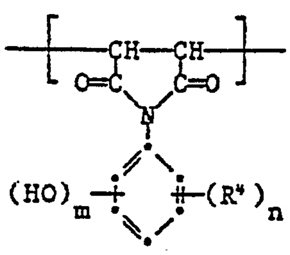

(II),

in which $R^4$ is $C_1$-$C_4$alkyl, $C_1$-$C_4$alkoxy or halogen, m is 1, 2 or 3, n is 0, 1, 2, 3 or 4 and the sum of m and n

is at most 5, it being possible for the radicals $R^4$ of a phenyl nucleus to be different within the definitions given.

5. A composition according to claim 4, in which binder iii) is a copolymer based on alkenylphenols and vinyl compounds with no carboxyl groups, or binder iii) is a copolymer based on N-hydroxyphenylmaleimide and vinyl compounds with no carboxyl groups.

6. A composition according to claim 4, containing 30-100 mol%, based on the total polymer, of structural units of the formula II and formula III, the proportion of radicals of the formula II, based on the total amount of II and III, making up 5 to 95 mol%

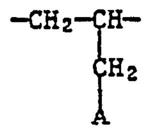 (III),

in which A is selected from the group of radicals consisting of halogen, cyano or structural units of the formulae IV to IX

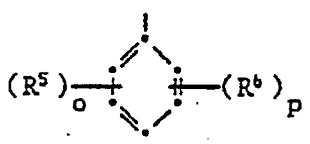 (IV), 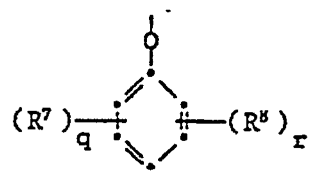 (V),

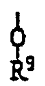 (VI), 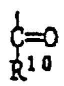 (VII), 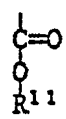 (VIII), 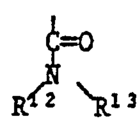 (IX),

in which $R^5$ and $R^7$ independently of one another are hydroxyl or glycidyl groups of the formula Xa or Xb

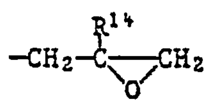 (Xa), 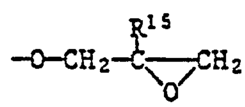 (Xb),

$R^6$ and $R^8$ independently of one another are $C_1$–$C_4$alkyl, $C_1$-$C_4$alkoxy or halogen,
o and q independently of one another are 0, 1, 2, or 3,
p and r independently of one another are 0, 1, 2, 3, 4 or 5, and the sums of o+p and q+r may be at most 5,
$R^9$ is hydrogen, $C_1$–$C_{20}$alkyl, a glycidyl radical of the formula Xa or a radical of the formula VII,
$R^{10}$ is hydrogen, $C_1$–$C_{20}$alkyl, cycloalkyl with 5 to 7 ring C atoms, phenyl, naphthyl or benzyl,
$R^{11}$ is hydrogen, $C_1$–$C_{20}$alkyl or a glycidyl radical of the formula Xa and the groups $R^{12}$ and $R^{13}$ independently of one another are hydrogen, $C_1$–$C_{20}$alkyl, cycloalkyl with 5 to 7 ring C atoms, substituted or unsubstituted aryl or aralkyl or a glycidyl radical of the formula Xa, or, together with the common nitrogen atom, form a five- or six-membered heterocyclic ring, and $R^{14}$ and $R^{15}$ independently of one another are hydrogen or methyl, it being possible for the radicals $R^5$ to $R^{15}$ and A of a polymer molecule to be different within the definitions given.

7. A composition according to claim 6, containing copolymers with 50-100 mol%, based on the total polymer, of structural units of the formulae II and III.

8. A composition according to claim 6, in which the binder essentially consists of structural elements of the formulae II and III, the proportion of elements II making up 10 – 18 mol%, based on the amount of II and III.

9. A composition according to claim 6, in which A is selected from the group comprising radicals of the formulae IV, V, VI, VIII an IX, $R^5$ and $R^7$ are glycidyl groups of the formulae Xa or Xb, $R^9$, $R^{11}$ and at least one of the radicals $R^{12}$ or $R^{13}$ is a glycidyl group of the formula Xa and o and q independently of one another are 1, 2 or 3.

10. A composition according to claim 6, in which A is a group of the formula VI and $R^9$ is a group of the formula Xa, or in which A is a group of the formulae IV or V, $R^5$ and $R^7$ are glycidyl groups of the formulae Xb, o and q are 1 or 2 and p and r are 0.

11. A composition according to claim 6, containing copolymers with at least 50 mol%, based on the total polymer, of structural elements of the formulae II and III and, as the residual amount, structural elements which are derived from vinyl compounds with no carboxyl groups selected from the group consisting of styrene types, esters or amides of α,β-unsaturated acids, nitriles of α,β-unsaturated acids, halogen-containing vinyl compounds, vinyl esters and vinyl ethers.

12. A composition according to claim 1, in which component b) is derived from a polyglycidyl ether of a novolak formed by reaction of formaldehyde with a phenol, diglycidyl ethers of aliphatic diols and cycloaliphatic epoxy resins.

13. A composition according to claim 1, in which component b) is a polyglycidyl compound which can be dispersed in water in the non-cured state.

14. A composition according to claim 13, in which component b) is a polyglycidyl ether of tetramethylolcyclohexanol or -cyclohexanone or a di- or triglycidyl glycerol ether.

15. A composition according to claim 1, in which the photoinitiator c) is selected from the compounds of the formulae XI, XII and XIII

$$\left[ R^{16}_{h} — I — R^{17}_{j} \right]^{\oplus} \left[ LQ_{m} \right]^{\ominus} \quad (XI),$$

$$\left[ R^{18} — \overset{\overset{O}{\|}}{I} — R^{19} \right]^{\oplus} \left[ LQ_{m} \right]^{\ominus} \quad (XII),$$

$$\left[ R^{20} — \underset{\underset{R^{21}}{|}}{S} — R^{22} \right]^{\oplus} \left[ LQ_{m} \right]^{\ominus} \quad (XIII),$$

in which h is 1 and j is 1 or h is 2 and j is 0, and $R^{16}$ and $R^{17}$, where h = 1 and j = 1, independently of one another are monovalent carbocyclic-aromatic $C_6$–$C_{18}$ radicals which are unsubstituted or carry 1 to 3 substituents, and $R^{16}$, in the case where h = 2 and j = 0, is a divalent, carbocyclic-aromatic $C_{12}$–$C_{18}$ radical which is unsubstituted or carries 1 to 3 substituents $R^{18}$, $R^{19}$, $R^{20}$, $R^{21}$ and $R^{22}$ independently of one another assume one of the meanings of $R^{17}$, L is selected from the group consisting of B, P, As and Sb, Q is a halogen atom, or some of the radicals Q in one anion $LQ_m^-$ can also be hydroxyl groups, and m is an integer which corresponds to the valency of L increased by one.

16. A composition according to claim 1, in which the photoinitiator c) is a compound of the formula XIV

$$\left[ R^{23}(Fe^{II}R^{24})_{s} \right]^{+s}_{t} \left[ X \right]^{-t}_{s} \quad (XIV),$$

in which s is 1 or 2, t is 1, 2, 3, 4 or 5, X is a non-nucleophilic anion, especially $PF_6^-$, $AsF_6^-$, $SbF_6^-$, $CF_3SO_3^-$, $C_2F_5SO_3^-$, n-$C_3F_7SO_3^-$, n-$C_4F_9SO_3^-$ and n-$C_8F_{17}SO_3^-$, $R^{23}$ is a π-arene and $R^{24}$ is an anion of a π-arene, especially a cyclopentadienyl anion.

17. A process for the production of relief structures comprising the following operating steps:
a) coating of a substrate with a radiation-sensitive composition according to claim 1,
b) exposure of the coated substrate with a given pattern of actinic radiation,
c) if appropriate, after-treatment with heat,
d) a development stage and
e) if appropriate, after-treatment of the developed system with heat.

18. A process for the production of protective coatings comprising the following operating steps:
a) coating of a substrate with a radiation-sensitive composition according to claim 1,
b) exposure of the radiation-sensitive coating over its entire surface and
c) if appropriate after-treatment with heat.

19. The use of the composition according to claim 1 for the production of protective coatings and relief structures.

**Revendications**

1. Composition de photoréserve en négatif développable en milieu alcalin aqueux, qui est essentiellement formée de:

a) un ou plusieurs polyphénols filmogènes solides ayant suffisamment d'hydroxyles phénoliques pour assurer le développement ou le gonflement dans une solution hydroalcaline d'un développateur,

b) un ou plusieurs composés ayant par molécule au moins deux groupes époxy ou au moins deux groupes d'éthers vinyliques ou encore au moins un groupe époxy et un groupe d'éther vinylique,

c) un ou plusieurs sels d'oniums à anions peu nucléophiles ou bien un ou plusieurs sels de métallocènes comme photo-inducteurs cationiques pour le composant b), et

d) le cas échéant des additifs courants.

2. Composition selon la revendication 1 dont le composant b) est constitué d'un ou de plusieurs composés ayant au moins deux groupes époxy par molécule.

3. Composition selon la revendication 1 dont le liant a) est pris parmi les suivants:

1) Novolaques d'un ou de plusieurs phénols et d'un ou de plusieurs aldéhydes,

2) Homopolymères et copolymères d'alcénylphénols, et

3) Homopolymères et copolymères de N-hydroxyphénylmaléimides.

4. Composition selon la revendication 3 dont la novolaque 1 provient d'un phénol bicyclique ou monocyclique éventuellement substitué et d'un aldéhyde en $C_1$–$C_6$, ou bien l'homopolymère ou le copolymère des alcénylphénols 2 comprend le motif de formule ci-dessous

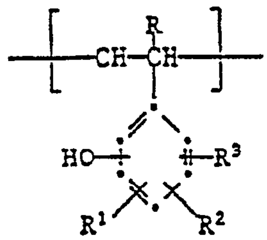

(I),

(R désignant l'hydrogène ou le groupe méthyle et $R^1$, $R^2$, et $R^3$ sont chacun, indépendamment les uns des autres, l'hydrogène, un alkyle ou un alcoxy en $C_1$–$C_4$, un halogène ou le groupe méthylol), ou encore l'homopolymère ou le copolymère du N-hydroxyphényl-maléimide 3 comporte le motif de formule II ci-dessous

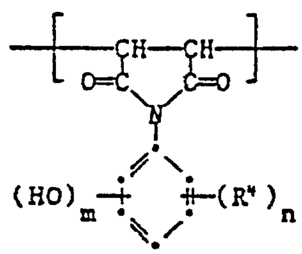

(II),

dans laquelle $R^4$ est un alkyle ou un alcoxy en $C_1$–$C_4$ ou un halogène, m le nombre 1, 2 ou 3, n le nombre 0, 1, 2, 3 ou 4 et la somme m + n ne dépasse pas 5, les radicaux $R^4$ d'un même cycle phénylique pouvant différer les uns des autres dans le cadre de leurs définitions indiquées.

5. Composition selon la revendication 4 dont le liant 3 est un copolymère à base d'alcénylphénols et de composés vinyliques sans groupes carboxyliques, ou bien ce liant 3 est un copolymère à base de N-hydroxyphényl-maléimide et de composés vinyliques sans groupes carboxyliques.

6. Composition selon la revendication 4 qui comprend de 30 à 100 Mol%, par rapport à l'ensemble du polymère, de motifs de formule II et de formule III ci-dessous, la proportion des motifs II par rapport au total de II et III étant de 5 à 95 Mol%:

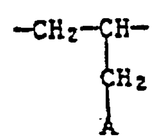

(III),

A étant pris parmi les halogènes, le groupe cyano et les motifs de formules IV à IX ci-après

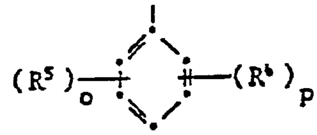 (IV), 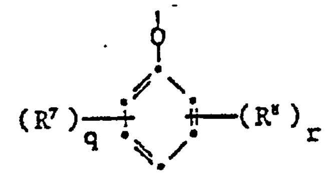 (V),

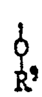 (VI), 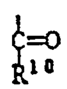 (VII), 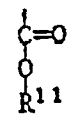 (VIII), 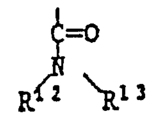 (IX),

dans lesquels $R^5$ et $R^7$ désignent indépendamment les uns des autres des groupes hydroxyliques ou glycidyliques de formules Xa et Xb:

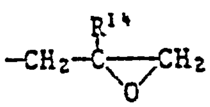 (Xa), 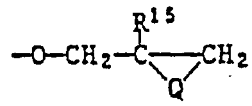 (Xb),

$R^6$ et $R^8$ des alkyles ou alcoxy en $C_1$-$C_4$ ou des halogènes,
o et q sont chacun, indépendamment l'un de l'autre, le nombre 0, 1, 2 ou 3,
p et r les nombres 0, 1, 2, 3, 4 ou 5, les sommes o + p et q + r ne devant pas dépasser 5 chacune,
$R^9$ désigne l'hydrogène, un alkyle en $C_1$–$C_{20}$, un radical glycidylique Xa ou un radical de formule VII,
$R^{10}$ l'hydrogène, un alkyle en $C_{1-20}$, un cycloalkyle avec de 5 à 7 atomes de carbone dans le cycle, ou encore le radical phényle, naphtyle ou benzyle,
$R^{11}$ l'hydrogène, un alkyle en $C_1$–$C_{20}$ ou un glycidyle Xa, et $R^{12}$ et $R^{13}$ sont chacun indépendamment l'un de l'autre l'hydrogène, un alkyle en $C_1$-$C_{20}$, un cycloalkyle avec de 5 à 7 atomes de carbone dans le cycle, un aryle ou un aralkyle éventuellement substitués ou encore un glycidyle de formule Xa, ou bien $R^{12}$ et $R^{13}$ forment ensemble et avec l'atome d'azote un hétérocycle pentagonal ou hexagonal, $R^{14}$ et $R^{15}$ sont chacun indépendamment l'un de l'autre l'hydrogène ou le groupe méthyle, les radicaux $R^5$ à $R^{15}$ et A d'une même molécule pouvant être différents les uns des autres dans le cadre de leurs définitions.

7. Composition selon la revendication 6 dont le copolymère comporte de 50 à 100 Mol% du total de motifs de formules II et III.

8. Composition selon la revendication 6 dont le liant est essentiellement formé de motifs II et III, avec une proportion de motifs II de l0 à 80 Mol% par rapport à la somme de II et III.

9. Composition selon la revendication 6 dans laquelle A est choisi parmi les radicaux de formules IV, V, VI, VIII et IX, $R^5$ et $R^7$ sont des groupes glycidyliques de formule Xa ou Xb, $R^9$, $R^{11}$ et l'un au moins des radicaux $R^{12}$ et $R^{13}$ sont des groupes glycidyliques de formule Xa et o et q sont le nombre 1, 2 ou 3 indépendamment l'un de l'autre.

10. Composition selon la revendication 6 dans laquelle A est un groupe de formule VI et $R^9$ un groupe de formule Xa, ou bien A est un groupe de formule IV ou V, $R^5$ et $R^7$ sont des groupes glycidyliques de formule Xb, o et q sont chacun le nombre 1 ou 2 et p et r le nombre 0.

11. Composition selon la revendication 6 qui comprend un copolymère avec au moins 50 Mol% de motifs de formules II et III et dont les autres motifs proviennent de composés vinyliques sans groupes carboxyliques choisis parmi le styrène et des dérivés du styrène, des esters, amides et nitriles d'acides α, β-insaturés, des composés vinyliques halogénés et des esters et éthers vinyliques.

12. Composition selon la revendication 1 dont le composant b) dérive d'un éther polyglycidylique d'une novolaque formée par réaction du formaldéhyde avec un phénol, d'éthers diglycidyliques de diols aliphatiques et de résines époxydes cycloaliphatiques.

13. Composition selon la revendication 1 dont le composant b) est un composé polyglycidylique pouvant être dispersé dans l'eau à l'état non-durci.

14. Composition selon la revendication 13 dont le composant b) est un éther polyglycidylique du tétraméthylolcyclolhexanol ou de la tétraméthylolhexanone ou un éther diglycidylique ou triglycidylique du glycérol.

15. Composition selon la revendication 1 dans laquelle le photo-inducteur c) est choisi parmi les composés de formules XI, XII et XIII ci-dessous

$$\left[ R^{16}_{h} - I - R^{17}_{j} \right]^{\oplus} \left[ LQ_{m} \right]^{\ominus} \quad (XI),$$

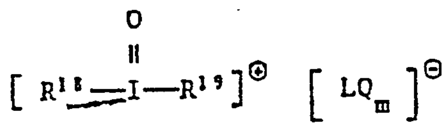

(XII),

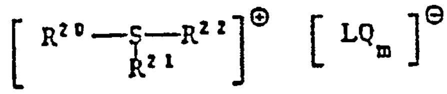

(XIII),

formules dans lesquelles h = 1 et j = 1 ou bien h = 2 et j = 0, si h = 1 et j = 1, $R^{16}$ et $R^{17}$ sont indépendamment l'un de l'autre des radicaux carbocyclo-aromatiques monovalents en $C_6$–$C_{18}$, avec éventuellement de un à trois substituants, alors que si h = 2 et j = 0 $R^{16}$ est un radical carbocyclo-aromatique bivalent en $C_{12}$–$C_{18}$ avec aussi éventuellement de un à trois substituants, $R^{18}$, $R^{19}$, $R^{20}$, $R^{21}$ et $R^{22}$ ont indépendamment les uns des autres l'une des significations de $R^{17}$, L est prls parmi B, P, As et Sb, Q est un atome halogène ou bien certains des radicaux Q d'un anion $LQ_m^-$ peuvent être aussi des hydroxyles, et m est un entier qui correspond à la valence de L élevée de 1.

16. Composition selon la revendication 1 dans laquelle le photo-inducteur c) est un composé de formule XIV

$$\left[ R^{23}(Fe^{II}R^{24})_{s} \right]^{+s}_{t} \left[ X \right]^{-t}_{s} \quad (XIV),$$

formule dans laquelle s = 1 ou 2, t = 1, 2, 3, 4 ou 5, X représente un anion non nucléophile, en particulier l'anion $PF_6$, $SbF_6$, $CF_3SO_3$, $C_2F_5SO_3$, n–$C_3F_7SO_3$, n-$C_4F_9SO_3$ ou n-$C_8F_{17}SO_3$, $R^{23}$ un $\pi$-arène et $R^{24}$ l'anion d'un $\pi$-arène, notamment un anion cyclopentadiényle.

17. Procédé de production de structures en relief qui comprend les opérations suivantes:

a) Revêtement d'un substrat d'une composition sensible aux rayonnements selon la revendication 1,

b) Irradiation du substrat recouvert par un rayonnement actinique conformément à un modèle prédéterminé, éventuellement

c) Traitement thermique,

d) Développement et le cas échéant

e) Traitement-thermique du matériau développé.

18. Procédé d'une formation de couches protectrices qui comprend les opérations suivantes:

a) Revêtement d'un substrat d'une composition sensible aux rayonnements selon la revendication 1,

b) Irradiation de la couche appliquée sur toute sa surface et,

c) le cas échéant traitement thermique de la couche irradiée.

19. Emploi d'une composition selon la revendication 1 pour former des couches protectrices ou des structures en relief.